(19) Europäisches Patentamt
European Patent Office
Office européen des brevets

(11) Publication number: **0 053 011**
**B1**

# (12) EUROPEAN PATENT SPECIFICATION

(45) Date of publication of patent specification: **03.04.85**

(21) Application number: **81305475.6**

(22) Date of filing: **19.11.81**

(51) Int. Cl.[4]: **C 07 D 213/82,**
C 07 D 213/83, A 01 N 43/40
// C07D213/80, C07C87/52,
C07C143/44

## (54) New herbicidal nicotinamide derivatives.

(30) Priority: **21.11.80 GB 8037372**

(43) Date of publication of application:
**02.06.82 Bulletin 82/22**

(45) Publication of the grant of the patent:
**03.04.85 Bulletin 85/14**

(84) Designated Contracting States:
**AT BE CH DE FR GB IT LI LU NL SE**

(56) References cited:
**FR-A-2 197 872**
**GB-A-1 550 574**
**US-A-4 270 946**

(73) Proprietor: **MAY & BAKER LIMITED**
**Dagenham Essex, RM10 7XS (GB)**

(72) Inventor: **Cramp, Michael Colin**
**63 Eastern Road**
**Romford Essex (GB)**
Inventor: **Gilmour, James**
**7 Baron Gardens**
**Barkingside Essex (GB)**
Inventor: **Parnell, Edgar William**
**10 Brookside Emerson Park**
**Hornchurch Essex (GB)**

(74) Representative: **Bentham, Stephen et al**
**J.A. Kemp & Co. 14 South Square Gray's Inn**
**London WC1R 5EU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 053 011 B1

**Description**

This invention relates to new nicotinamide derivatives, to processes for their preparation, to compositions containing them and to their use in agriculture as pre- and post-emergence herbicides.

As a result of research and experimentation, it has been found that the new nicotinamide derivatives of the general formula:—

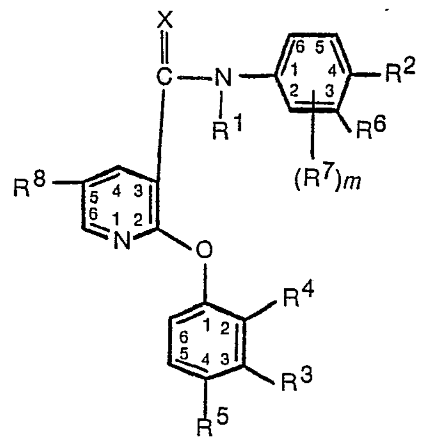

(I)

wherein X represents an oxygen or sulphur atom, $R^1$ represents a hydrogen atom or a methyl or ethyl group, $R^2$ represents a hydrogen or fluorine atom, a cyano group or a methyl or ethyl group optionally substituted by one or more fluorine atoms (preferably trifluoromethyl), $R^3$ represents a fluorine, chlorine or bromine atom or a cyano, trifluoromethyl, methoxy, ethoxy, trifluoromethyoxy or akanesulphonyl group, wherein the alkane moiety of the alkanesulphonyl group may be straight- or branched-chain and contains from 1 to 4 carbon atoms (e.g. methanesulphonyl or ethanesulphonyl), one of $R^4$ and $R^5$ represents a hydrogen atom and the other represents a hydrogen, fluorine, chlorine or bromine atom or a cyano, trifluoromethyl, methoxy, ethoxy, trifluoromethoxy or akanesulphonyl group, wherein the alkane moiety of the alkanesulphonyl group may be straight- or branched-chain and contains from 1 to 4 carbon atoms (e.g. methanesulphonyl or ethanesulphonyl), $R^6$ represents a hydrogen, fluorine or chlorine atom, $R^7$ represents a fluorine or chlorine atom, $R^8$ represents a hydrogen, fluorine, chlorine or bromine atom or a methyl or ethyl group and *m* represents 0, 1 or 2, wherein, when *m* represents 2, the atoms represented by the symbol $R^7$ may be the same or different, with the provisos (1) that when $R^2$ represents a hydrogen atom, $R^6$ represents a hydrogen atom and *m* represents 0, and (2) that when $R^2$ represents a fluorine atom, a cyano group or a methyl or ethyl group optionally substituted by one or more fluorine atoms, and $R^6$ represents a fluorine or chlorine atom, *m* represents 0 or 1, possess valuable herbicidal properties. Preferred compounds are those wherein $R^2$ represents a hydrogen or fluorine atom, a cyano group, or a methyl or trifluoromethyl group, $R^3$ represents a methoxy group or, more especially, a fluorine, chlorine or bromine atom or a cyano, trifluoromethyl, methanesulphonyl or ethanesulphonyl group, one of $R^4$ and $R^5$ represents a hydrogen atom and the other represents a fluorine or chlorine atom or, more especially, a hydrogen atom, R represents a chlorine or, more especially, fluorine atom, $R^8$ represents a hydrogen atom or a methyl group and X, $R^1$, $R^6$ and *m* are as hereinbefore defined; preferably $R^1$ represents a hydrogen atom or a methyl or ethyl group, and (a) $R^2$ represents a hydrogen atom, $R^6$ represents a hydrogen atom and *m* represents 0 or (b) $R^2$ represents a fluorine atom, a cyano group or a methyl or trifluoromethyl group, $R^6$ represents a hydrogen, chlorine or fluorine atom, $R^7$ represents a chlorine or fluorine atom and *m* represents 0 or 1.

Particularly preferred compounds are those wherein X represents a sulphur or, preferably, oxygen atom, $R^1$ represents a hydrogen atom or a methyl group, $R^2$ represents a hydrogen or fluorine atom, $R^3$ represents a trifluoromethyl group, $R^4$ and $R^5$ each represent a hydrogen atom, $R^6$ represents a hydrogen or fluorine atom, $R^7$ represents a fluorine atom, $R^8$ represents a hydrogen atom or a methyl group and *m* represents 0 or 1, with the provisos (i) that when $R^2$ represents a hydrogen atom, $R^6$ represents a hydrogen atom and *m* represents 0 and (ii) that when $R^2$ represents a fluorine atom, (a) $R^6$ represents a hydrogen atom and *m* represents 0 or *m* represents 1 and $R^7$ represents a fluorine atom in the 2-(or 6-) position of the phenyl ring, or (b) $R^6$ represents a fluorine atom and *m* represents 0, or *m* represents 1 and $R^7$ represents a fluorine atom in the 6- position of the phenyl ring; when $R^1$ represents a hydrogen atom $R^6$ preferably represents a hydrogen or fluorine atom. Those particularly preferred compounds in which $R^1$ represents a hydrogen atom have a high and persistent level of herbicidal activity against both dicotyledonous and monocotyledonous weeds; those particularly preferred compounds in which $R^1$ represents a methyl group have a level of herbicidal activity against dicotyledonous weeds similar to that exhibited by the particularly preferred compounds in which $R^1$ represents a hydrogen atom and a somewhat lower level of herbicidal activity against monocotyledonous weeds and are particularly useful in controlling the growth of

dicotyledonous weeds in cereal crops e.g. wheat. The herbicidal activity of the particularly preferred compounds in which $R^1$ represents a methyl group is also somewhat less persistent than that of the particularly preferred compounds in which $R^1$ represents a hydrogen atom. This lower persistence of herbicidal activity renders the particularly preferred compounds wherein $R^1$ represents a methyl group especially useful in controlling the growth of weeds, more particularly dicotyledonous weeds, in crop-growing areas on which crop-rotation is practised, where the persistence in the soil of a herbicide suitable for use in a given crop, may cause damage to a subsequent, different crop for use in which the herbicide is less suitable.

The following compounds of general formula I are of particular interest as herbicides:—

Compound No.
1 N-(4-fluorophenyl)-2-(3-trifluoromethylphenoxy)nicotinamide
2 N-phenyl-2-(3-trifluoromethylphenoxy)nicotinamide
3 N-(2,4-difluorophenyl-2-(3-trifluoromethylphenoxy)nicotinamide
4 N-(3-chloro-4-fluorophenyl)-2-(3-trifluoromethylphenoxy)nicotinamide
5 N-(4-methylphenyl)-2-(3-trifluoromethylphenoxy)nicotinamide
7 N-(2,4,6-trifluorophenyl)-2-(3-trifluoromethylphenoxy)nicotinamide
9 N-(3,4-difluorophenyl)-2-(3-trifluoromethylphenoxy)nicotinamide
12 N-(2,4,5-trifluorophenyl)-2-(3-trifluoromethylphenoxy)nicotinamide
13 N-(4-fluorophenyl)-N-methyl-2-(3-trifluoromethylphenoxy)nicotinamide
15 2-(3-fluorophenoxy)-N-(4-fluorophenyl)nicotinamide
16 2-(3-chlorophenoxy)-N-(4-fluorophenyl)nicotinamide
17 2-(2,3-dichlorophenoxy)-N-(4-fluorophenyl)nicotinamide
18 2-(3,4-dichlorophenoxy)-N-(4-fluorophenyl)nicotinamide
19 2-(3-bromophenoxy)-N-(4-fluorophenyl)nicotinamide
20 N-(4-fluorophenyl)-2-(3-methoxyphenoxy)nicotinamide
21 2-(3-cyanophenoxy)-N-(4-fluorophenyl)nicotinamide
22 N-(4-fluorophenyl)-2-(3-methanesulphonylphenoxy)nicotinamide
23 2-(3-chloro-4-fluorophenoxy)-N-(4-fluorophenyl)nicotinamide
24 2-(4-chloro-3-trifluoromethylphenoxy)-N-(4-fluorophenyl)nicotinamide
25 2-(3-ethanesulphonylphenoxy)-N-(4-fluorophenyl)nicotinamide
26 2-(3,4-difluorophenoxy)-N-(4-fluorophenyl)nicotinamide
27 N-(2,4-difluorophenyl)-2-(3-methanesulphonylphenoxy)nicotinamide
28 2-(3-bromophenoxy)-N-(2,4-difluorophenyl)nicotinamide
29 2-(3-chloro-4-fluorophenoxy)-N-(2,4-difluorophenyl)nicotinamide
30 2-(3-ethanesulphonylphenoxy)-N-(2,4-difluorophenyl)nicotinamide
31 2-(3-chlorophenoxy)-N-phenylnicotinamide
32 2-(3-chlorophenoxy)-N-(2,4-difluorophenyl)nicotinamide
33 2-(3-chlorophenoxy)-N-(2,4,6-trifluorophenyl)nicotinamide
34 N-(3-chloro-4-fluorophenyl)-2-(3-chlorophenoxy)nicotinamide
35 2-(3-chlorophenoxy)-N-(4-methylphenyl)nicotinamide
36 2-(3-chlorophenoxy)-N-(4-cyanophenyl)nicotinamide
38 2-(3-chlorophenoxy)-N-(4-fluorophenyl)-N-methyl-nicotinamide
39 2-(3-chlorophenoxy)-N-(2,4,5-trifluorophenyl)nicotinamide
41 2-(3-chlorophenoxy)-N-ethyl-N-(4-fluorophenyl)nicotinamide
42 2-(3-chlorophenoxy)-N-(4-trifluoromethylphenyl)nicotinamide
43 2-(3-chlorophenoxy)-N-(3,4-difluorophenyl)nicotinamide
44 2-(3-methoxyphenoxy)-N-phenylnicotinamide
45 N-(2,4-difluorophenyl)-2-(3-methoxyphenoxy)nicotinamide
46 2-(3-methoxyphenoxy)-N-(2,4,6-trifluorophenyl)nicotinamide
47 2-(3-methoxyphenoxy)-N-(4-methylphenyl)nicotinamide
48 N-(3-chloro-4-fluorophenyl)-2-(3-methoxyphenoxy)nicotinamide
49 2-(3-chlorophenoxy)-N-(4-fluorophenyl)thionicotinamide
50 N-(4-fluorophenyl)-2-(3-trifluoromethylphenoxy)thionicotinamide
51 N-(2,4-difluorophenyl)-2-(3-trifluoromethylphenoxy)thionicotinamide
52 N-(2,4-difluorophenyl)-2-(3-fluorophenoxy)nicotinamide
53 N-methyl-N-phenyl-2-(3-trifluoromethylphenoxy)nicotinamide
55 2-(4-chloro-3-trifluoromethylphenoxy)-N-(2,4-difluorophenyl)nicotinamide
56 2-(3-trifluoromethylphenoxy)-N-(2,4-difluorophenyl)-5-methylnicotinamide

especially Compounds Nos. 4, 5, 7, 15, 16, 17, 19, 20, 21, 22, 23, 24, 25, 26, 27, 28, 29, 30, 31, 32, 33, 34, 35, 38, 39, 41, 42, 43, 44, 49, 51 and 52 and, more especially, Compounds Nos. 1, 2, 3, 9, 12, 13, 50, 53 and 56.

Where, hereinafter, compounds are identified by number, these numbers identify individual compounds of general formula I as hereinbefore indicated.

According to a feature of the present invention, there is provided a method for controlling the growth of weeds (i.e. undesired vegetation) at a locus which comprises applying to the locus a herbicidally

effective amount of at least one nicotinamide derivative of general formula I. For this purpose, the nicotinamide derivatives are normally used in the form of herbicidal compositions (i.e. in association with compatible diluents or carriers suitable for use in herbicidal compositions), for example as hereinafter described.

The compounds of general formula I show herbicidal activity against dicotyledonous (i.e. broad-leafed) and monocotyledonous (e.g. grass) weeds by pre- and/or post-emergence application.

By the term "pre-emergence application" is meant application to the soil in which the weed seeds or seedlings are present before emergence of the weeds above the surface of the soil. By the term "post-emergence application" is meant application to the aerial or exposed portions of the weeds which have emerged above the surface of the soil. For example, the compounds of general formula I may be used to control the growth of broad-leafed weeds, for example, *Aethusa cynapium, Abutilon theophrasti, Amaranthus retroflexus, Amsinckia intermedia, Anagallis arvensis, Anthemis arvensis, Atriplex patula, Bidens pilosa, Brassica nigra, Capsella bursa-pastoris, Chenopodium album, Chrysanthemum segetum, Cirsium arvense, Datura stramonium, Desmodium tortuosum, Emex australis, Euphorbia helioscopia, Fumaria officinalis, Galeopsis tetrahit, Galium aparine, Geranium dissectum, Ipomea purpurea, Lamium purpureum, Lapsana communis, Matricaria inodora, Monochoria vaginalis, Papaver rhoeas, Physalis longifolia, Plantago lanceolata, Polygonum* spp., (e.g. *Polygonum aviculare, Polygonum convolvulus* and *Polygonum persicaria), Portulaca oleracea, Raphanus raphanistrum, Rotala indica, Rumex obtusifolius, Saponaria vaccaria, Scandix pecten-veneris, Senecio vulgaris, Sesbania florida, Sida spinosa, Silene alba, Sinapis arvensis, Solanum nigrum, Sonchus arvensis, Spergula arvensis, Stellaria media, Thlaspi arvense, Tribulus terrestria, Urtica urens, Veronica hederifolia, Veronica persica, Viola arvensis* and *Xanthium strumarium,* and grass weeds, for example, *Alopecurus myosuroides, Apera spica-venti, Agrostis stolonifera, Avena fatua, Avena ludoviciana, Brachiaria* spp., *Bromus sterilis, Bromus tectorum, Cenchrus* spp., *Cynodon dactylon, Digitaria sanquinalis, Echinochloa crus-galli, Eleusine indica, Setaria viridis* and *Sorghum halepense* and sedges, for example *Cyperus esculentus, Cyperus iria* and *Cyperus rotundus,* and *Eleocharis acicularis.*

The amounts of compounds of general formula I applied vary with the nature of the weeds, the compositions used, the time of application, the climatic and edaphic conditions and (when used to control the growth of weeds in crop-growing areas) the nature of the crops. When applied to a crop-growing area, the rate of application should be sufficient to control the growth of weeds without causing substantial permanent damage to the crop. In general, taking these factors into account, application rates between 0.1 kg and 20 kg of active material per hectare give good results. However, it is to be understood that higher or lower application rates may be used, depending upon the particular problem of weed control encountered.

The compounds of general formula I may be used to control selectively the growth of weeds, for example to control the growth of those species hereinbefore mentioned, by pre- or post-emergence application in a directional or non-directional fashion, e.g. by directional or non-directional spraying, to a lucus of weed infestation which is an area used, or to be used, for growing crops, for example cereals, e.g. wheat, barley, oats, maize and rice, soya beans, field and dwarf beans, peas, lucerne, cotton, peanuts, flax, onions, carrots, cabbage, oilseed rape, sunflower, sugar beet, and permanent or sown grassland before or after sowing of the crop or before or after emergence of the crop. For the selective control of weeds at a locus of weed infestation which is an area used, or to be used, for the growing of crops, e.g. the crops hereinbefore mentioned, application rates between 0.1 kg and 4.0 kg, and preferably between 0.2 kg and 2.0 kg, of active material per hextare are particularly suitable. More particularly, the compounds of general formula I may be used to control selectively the growth of broad leafed weeds, for example to control the growth of those broad leafed species hereinbefore mentioned, by pre- or post-emergence application, and more especially pre-emergence application, in a non-directional fashion, e.g. by non-directional spraying, to an area used for growing cereal crops before or after emergence of both the crop and weeds.

For this purpose, i.e. the selective control of broad leafed weeds by pre- or post-emergence application to an area used for growing cereal crops, application rates between 0.1 and 4.0 kg, and preferably between 0.2 kg and 2.0 kg, of active material per hectare are particularly suitable.

The compounds of general formula I may also be used to control the growth of weeds, especially those indicated above, by pre- or post-emergence application in established orchards and other tree-growing areas, for example forests, woods and parks, and plantations, e.g. sugar cane, oil palm and rubber plantations. For this purpose they may be applied in a directional or non-directional fashion (e.g. by directional or non-directional spraying) to the weeds or to the soil in which they are expected to appear, before or after planting of the trees or plantations at application rates between 0.5 kg and 10.0 kg, and preferably between 1.0 kg and 4.0 kg, of active material per hectare.

The compounds of general formula I may also be used to control the growth of weeds, especially those indicated above, at loci which are not crop-growing areas but in which the control of weeds is nevertheless desirable. Examples of such non-crop-growing areas include airfields, industrial sites, railways, roadside verges, the verges of rivers, irrigation and other waterways, scrublands and fallow or uncultivated land, in particular where it is desired to control the growth of weeds in order to reduce fire risks. When used for such purposes in which a total herbicidal effect is frequently desired, the active compounds are normally applied at dosage rates higher than those used in crop-growing areas as hereinbefore described. The

precise dosage will depend upon the nature of the vegetation treated and the effect sought. Pre- or post-emergence application, and preferably pre-emergence application, in a directional or non-directional fashion (e.g. by directional or non-directional spraying) at application rates between 2.0 kg and 20.0 kg, and preferably between 4.0 and 10.0 kg, of active material per hectare are particularly suitable for this purpose.

When used to control the growth of weeds by pre-emergence application, the compounds of general formula I may be incorporated into the soil in which the weeds are expected to emerge. It will be appreciated that when the compounds of general formula I are used to control the growth of weeds by post-emergence application, i.e. by application to the aerial or exposed portions of emerged weeds, the compounds of general formula I will also normally come into contact with the soil and may also then exercise a pre-emergence control on later-germinating weeds in the soil.

Where especially prolonged weed control is required, the application of the compounds of general formula I may be repeated if required.

According to a further feature of the present invention, there are provided compositions suitable for herbicidal use comprising one or more of the nicotinamide derivatives of general formula I in association with, and preferably homogeneously dispersed in, one or more compatible herbicidally-acceptable diluents or carriers (i.e. diluents or carriers of the type generally accepted in the art as being suitable for use in herbicidal compositions and which are compatible with compounds of general formula I). The term "homogeneously dispersed" is used to include compositions in which the compounds of general formula I are dissolved in the other components. The term "herbicidal compositions" is used in a broad sense to include not only compositions which are ready for use as herbicides but also concentrates which must be diluted before use. Preferably, the compositions contain from 0.05 to 90% by weight of one or more compounds of general formula I.

The herbicidal compositions may contain both a diluent or carrier and a surface-active (e.g. wetting, dispersing, or emulsifying) agent. Surface-active agents which may be present in herbicidal compositions of the present invention may be of the ionic or non-ionic types, for example sulphoricinoleates, quaternary ammonium derivatives, products based on condensates of ethylene oxide with nonyl- or octylphenols, or carboxylic acid esters of anhydrosorbitols which have been rendered soluble by etherification of the free hydroxy groups by condensation with ethylene oxide, alkali and alkaline earth metal salts of sulphuric acid esters and sulphonic acids such as dinonyl- and dioctyl-sodium sulphonosuccinates and alkali and alkaline earth metal salts of high molecular weight sulphonic acid derivatives such as sodium and calcium lignosulphonates.

Suitably, herbicidal compositions according to the present invention may comprise from 0.05% to 10% of surface-active agent but, if desired, herbicidal compositions according to the present invention may comprise higher proportions of surface-active agent, for example up to 15% in liquid emulsifiable suspension concentrates and up to 25% in liquid water soluble concentrates.

Examples of suitable solid diluents or carriers are aluminium silicate, talc, calcined magnesia, kieselguhr, tricalcium phosphate, powdered cork, absorbent carbon black and clays such as kaolin and bentonite. The solid compositions (which may take the form of dusts, granules or wettable powders) are preferably prepared by grinding the compounds of general formula I with solid diluents or by impregnating the solid diluents or carriers with solutions of the compounds of general formula I in volatile solvents, evaporating the solvents and, if necessary, grinding the products so as to obtain powders. Granular formations may be prepared by absorbing the compounds of general formula I (dissolved in volatile solvents) onto the solid diluents or carriers in granular form and evaporating the solvents, or by granulating compositions in powder form obtained as described above. Solid herbicidal compositions, particularly wettable powders, may contain wetting or dispersing agent (for example of the types described above), which may also, when solid, serve as diluents or carriers.

Liquid compositions according to the invention may take the form of aqueous, organic or aqueous-organic solutions, suspensions and emulsions which may incorporate a surface-active agent. Suitable liquid diluents for incorporation in the liquid compositions include water, acetophenone, cyclohexanone, isophorone, toluene, xylene and mineral, animal and vegetable oils (and mixtures of these diluents). Surface-active agents, which may be present in the liquid compositions, may be ionic or non-ionic (for example of the types described above) and may, when liquid, also serve as diluents or carriers.

Wettable powders and liquid compositions in the form of concentrates may be diluted with water or other suitable diluents, for example mineral or vegetable oils, particularly in the case of liquid concentrates in which the diluent or carrier is an oil, to give compositions ready for use. When desired, liquid compositions of the compound of general formula I may be used in the form of self-emulsifying concentrates containing the active substances dissolved in the emulsifying agents or in solvents containing emulsifying agents compatible with the active substances, the simple addition of water to such concentrates producing compositions ready for use.

Liquid concentrates in which the diluent or carrier is an oil may be used without further dilution using the electrostatic spray technique.

Herbicidal compositions according to the present invention may also contain, if desired, conventional adjuvants such as adhesives, protective colloids, thickeners, penetrating agents, stabilisers, sequestering agents, anti-caking agents, colouring agents and corrosion inhibitors. These adjuvants may also serve as carriers or diluents.

Preferred herbicidal compositions according to the present invention are aqueous suspension concentrates which comprise from 10 to 70% w/v of one or more compounds of general formula I, from 2 to 10% w/v of surface-active agent, from 0.1 to 5% w/v of thickener and from 15 to 87.9% by volume of water; wettable powders which comprise from 10 to 90% w/w of one or more compounds of general formula I, from 2 to 10% w/w of surface-active agent and from 10 to 88% w/w of solid diluent or carrier; liquid water soluble concentrates which comprise from 10 to 30% w/v of one or more compounds of general formula I, from 5 to 25% w/v of surface-active agent and from 45 to 85% by volume of water-miscible solvent, e.g. dimethylformamide; liquid emulsifiable suspension concentrates which comprise from 10 to 70% w/v of one or more compounds of general formula I, from 5 to 15% w/v of surface-active agent, from 0.1 to 5% w/v of thickener and from 10 to 84.9% by volume of organic solvent; granules which comprise from 2 to 10% w/w of one or more compounds of general formula I, from 0.5 to 2% w/w of surface-active agent and from 88 to 97.5% w/w of granular carrier and emulsifiable concentrates which comprise from 0.05 to 90% w/v, and preferably from 1 to 60% w/v, of one or more compounds of general formula I, from 0.01 to 10% w/v, and preferably from 1 to 10% w/v, of surface-active agent and from 9.99 to 99.94%, and preferably from 39 to 98.99%, by volume of organic solvent.

Herbicidal compositions according to the present invention may also comprise the compounds of general formula I in association with, and preferably homogeneously dispersed in, one or more other pesticidally active compounds and, if desired, one or more compatible pesticidally acceptable diluents or carriers, surface-active agents and conventional adjuvants as hereinbefore described. Examples of other pesticidally active compounds which may be included in, or used in conjunction with, the herbicidal compositions of the present invention, include herbicides, for example to increase the range of weed species controlled, for example alachlor [α-chloro-2,6-diethyl-N-(methoxymethyl)acetanilide], asulam [methyl(4-aminobenzenesulphonyl)-carbamate], alloxydim Na [sodium salt of 2 - (1 - allyloxyaminobutylidene) - 5,5 - dimethyl - 4 - methoxycarbonylcyclohexane - 1,3 - dione], atrazine [2 - chloro - 4 - ethylamino - 6 - isopropylamino - 1,3,5 - triazine], barban [4 - chlorobut - 2 - ynyl N - (3 - chlorophenyl)carbamate], benzoylprop - ethyl [ethyl N - benzoyl - N - (3,4 - dichlorophenyl - 2 - amino-propionate], bromoxynil [3,5 - dibromo - 4 - hydroxybenzonitrile], butachlor [N - (butoxymethyl) - α - chloro - 2,6 - diethylacetanilide], butylate [S-ethyl N,N - diisobutyl(thiocarbamate)], carbetamide [D - N - ethyl - 2 - (phenylcarbamoyloxy)propionamide], chlorfenpropmethyl [methyl 2 - chloro - 3 - (4 - chlorophenyl)propionate], chlorpropham [isopropyl) N - (3 - chlorophenyl)carbamate], chlortoluron [N' - (3 - chloro - 4 - methylphenyl) - N,N - dimethylurea], cyanazine [2 - chloro - 4 - (1 - cyano - 1 - methylethylamino) - 6 - ethylamino - 1,3,5 - triazine], cycloate [N' - cyclohexy; - N - ethyl - S - ethyl-(thiocarbamate)], 2,4 - D[2,4 - dichlorophenoxyacetic acid], dalapon [2,2 - dichloropropionic acid], 2,4 - DB [4 - (2,4 - dichlorophenoxy)butyric acid], desmedipham [3 - (ethoxycarbonylamino)phenyl N - phenyl - carbamate], diallate [S - 2,3 - dichloroallyl - N,N - di - isopropyl(thiocarbamate)], dicamba [3,6 - dichloro - 2 - methoxybenzoic acid], dichlorprop [(±) - 2 - (2,4 - dichlorophenoxy)propionic acid], difenzoquat [1,2 - dimethyl - 3,5 - diphenyl - pyrazolium salts], dimefuron {4 - [2 - chloro - 4 - (3,3 - dimethylureido)phenyl] - 2 - t - butyl - 1,3,4 - oxadiazolin - 5 - one}, dinitramine [$N^1$,$N^1$ - diethyl - 2,6 - dinitro - 4 - trifluoromethyl - *m* - phenylenediamine], diuron [N' - (3,4 - dichlorophenyl) - N,N - dimethylurea], EPTC [S - ethyl N,N - dipropyl(thiocarbamate)], ethofumesate [2 - ethoxy - 2,3 - dihydro - 3,3 - dimethylbenzofuran - 5 - yl methylsulphonate], flampropisopropyl [isopropyl (±) - 2 - (N - benzoyl - 3 - chlor - 4 - fluoroanilino)propionate], flampropmethyl [methyl (±) - 2 - (N - benzoyl - 3 - chloro - 4 - fluoroanilino)propionate], fluometuron [N' - (3 - trifluoromethylphenyl) - N,N - dimethylurea], ioxynil [4 - hydroxy - 3,5 - di - iodobenzonitrile], isoproturon [N' - (4 - isopropylphenyl) - N,N - dimethylurea], linuron [N - (3,4 - dichlorophenyl) - N - methoxy - N - methylurea], MCPA [4 - chloro - 2 - methylphenoxyacetic acid], MCPB [4 - (4 - chloro - 2 - methylphenoxy)butyric acid], mecoprop[(±) - 2 - (4 - chloro - 2 - methylphenoxy)propionic acid], metamitron [4 - amino - 3 - methyl - 6 - phenyl - 1,2,4 - triazin - 5(4H) - one], methabenzthiazuron [N - (benzothiazol - 2 - yl) - N,N' - dimethylurea], metribuzin [4 - amino - 6 - t - butyl - 3 - (methylthio) - 1,2,4 - triazin - 5(4H) - one], molinate [S - ethyl N,N - hexamethylene(thiocarbamate)], oxadiazon [3 - (2,4 - dichloro - 5 - isopropoxyphenyl) - 5 - t - butyl - 1,3,4 - oxadiazolin - 2 - one], paraquat [1,1' - dimethyl - 4,4' - bipyridylium salts], pebulate [S - propyl N - butyl - N - ethyl (thiocarbamate)], phenmedipham [3 - (methoxycarbonylamino) - phenyl N - (3 - methylphenyl)carbamate], prometryne [4,6 - bisisopropyl-amino - 2 - methylthio - 1,3,5 - triazine], propachlor [α - chloro - N - isopropylacetanilide], propanil [N - (3,4 - dichlorophenyl) - propionamide], propham [isopropyl N - phenylcarbamate], pyrazone [5 - amino - 4 - chloro - 2 - phenylpyridazin - 3(2H) - one], simazine [2 - chloro - 4,6 - bisethylamino - 1,3,5 - triazine], TCA (trichloroacetic acid), thiobencarb [S - (4 - chlorobenzyl) - N,N - diethylthiolcarbamate], tri - allate [S - 2,3,3 - trichloroallyl N,N - di - isopropyl (thiocarbamate)], and trifluralin [2,6 - dinitro - N,N - dipropyl - 4 - trifluoromethylaniline]; insecticides, e.g. carbaryl [naphth - 1 - yl N - methylcarbamate]; synthetic pyrethroids, e.g. permethrin and cypermethrin; and fungicides, e.g. 2,6 - dimethyl - 4 - tridecyl - morpholine, methyl N - (1 - butylcarbamoyl - benzimidazol - 2 - yl)carbamate, 1,2 - bis - (3 - methoxycarbonyl - 2 - thioureido)benzene, isopropyl 1 - carbamoyl - 3 - (3,5 - dichlorophenyl)-hydantoin and 1 - (4 - chloro - phenoxy) - 3,3 - dimethyl - 1 - (1,2,4 - triazol - 1 - yl) - butan - 2 - one. Other bioligically active materials which may be included in, or used in conjunction with, the herbicidal compositions of the present invention are plant growth regulators, e.g. succinamic acid, (2-chloroethyl)-trimethylamminium chloride and 2-chloroethane-phosphonic acid; or fertilizers, e.g. containing nitrogen, potassium and phosphorus and trace elements known to be essential to successful plant life, e.g. iron, magnesium, zinc, manganese, cobalt and copper.

Pesticidally active compounds and other bioligically active materials which may be included in, or used in conjunction with, the herbicidal compositions of the present invention, for example those hereinbefore mentioned, and which are acids, may, if desired, be utilized in the form of conventional derivatives, for example alkali metal and amine salts and esters.

The following Examples illustrate herbicidal compositions according to the present invention.

Example 1

A wettable powder was prepared from:—

| | |
|---|---|
| 2-(3-chlorophenoxy)-N-(4-fluorophenyl) nicotinamide | 50% w/w |
| Pentrone T Powder (sulphated fatty alcohol) | 5% w/w |
| Clarcelflo SAS 132 (silicaceous carrier, predominantly aluminium silicate) | 45% w/w |

by intimately mixing the compound of general formula I with the Pentrone T powder and Clarcelflo SAS 132 in a blender, and then passing the resultant mix through a hammer-mill using a 0.5 mm screen. The wettable powder was then sieved through a 150 μ screen to remove coarse particles. The resulting formulation was then mixed with water at rate of 2 kg of wettable powder in 200 litres of water and applied as a spray to one hectare of soil sown with winter wheat, before the emergence of the weeds or crop to selectively control the growth of *Alopecurus myosuroides, Galium aparine, Veronica persica* and *Stellaria media.*

Example 2

N-(4-Fluorophenyl)-2-(3-trifluoromethylphenoxy) nicotinamide was formulated as a water soluble concentrate containing:—

| | |
|---|---|
| N-(4-fluorophenyl)-2-(3-trifluoromethylphenoxy) nicotinamide | 10% w/v (weight/volume) |
| Ethylan KEO (nonylphenyl/ethylene oxide condensate containing 9—10 moles of ethylene oxide per mol of phenol) | 10% w/v |
| Dimethylformamide | to 100% by volume, |

by dissolving the Ethylan KEO in a portion of dimethylformamide and then adding the active ingredient with heating and stirring until dissolved. The resulting solution was then made up to 100% by volume by adding the rest of the dimethylformamide.

5 Litres of the above formulation may be dissolved in 200 litres of water and sprayed pre-emergence onto 1 hectare of ground sown with a crop of winter-sown wheat to control *Stellaria media, Veronica persica, Galium aparine, Alopecurus myosuroides,* and *Matricaria inodora.*

The N-(4-fluorophenyl)-2-(3-trifluoromethylphenoxy)nicotinamide may, if desired, be replaced in the above water soluble concentrate by any other compound of general formula I.

Example 3

A wettable powder was formed from:—

| | |
|---|---|
| N-(4-fluorophenyl)-2-(3-trifluoromethylphenoxy)-nicotinamide | 50% w/w |
| Ethylan BCP (a nonylphenol/ethylene oxide condensate containing 9 moles of ethylene oxide per mol of phenol) | (weight/weight) 5% w/w |
| Aerosil (silicon dioxide of microfine particle size) | 5% w/w |
| Celite PF (synthetic magnesium silicate carrier) | 40% w/w |

by adsorbing the Ethylan BCP onto the Aerosil, mixing with the other ingredients and grinding the mixture in a hammer-mill to give a wettable powder which may be diluted with water and applied at an application rate of 1.0 kg of wettable powder in 300 litres of spray fluid per hectare to control the growth of *Galium aparine, Veronica persica, Viola arvensis,* and *Stellaria media* by pre-emergence application in a crop of winter wheat.

Similar wettable powders may be prepared as described above by replacing the N-(4-fluorophenyl)-2-(3-trifluoromethylphenoxy)nicotinamide by other compounds of general formula I.

Example 4

An aqueous suspension concentrate was formed from:—

| | |
|---|---|
| N-(4-fluorophenyl)-2-(3-trifluoromethylphenoxy)-nicotinamide | 50% w/v |
| Ethylan BCP | 1.0% w/v |
| Sopropon T36 (sodium salt of polycarboxylic acid) | 0.2% w/v |
| Ethylene glycol | 5% w/v |
| Rhodigel 23 (polysaccharide xanthan gum thickener) | 0.15 w/v |
| distilled water | to 100% by volume |

by intimately mixing the ingredients and grinding in a ball-mill for 24 hours. The concentrate thus obtained may be dispersed in water and applied at an application rate of 2.0 kg of aqueous suspension concentrate in 300 litres of spray fluid per hectare to control the growth of *Galium aparine, Veronica persica,* and *Stellaria media* by post-emergence application in an emerged crop of winter barley.

Similar aqueous suspension concentrates may be prepared as described above by replacing the N-(4-fluorophenyl)-2-(3-trifluoromethylphenoxy)nicotinamide by other compounds of general formula I.

Example 5

An emulsifiable suspension concentrate was formed from:—

| | |
|---|---|
| N-(4-fluorophenyl)-2-(3-trifluoromethylphenoxy) nicotinamide | 50% w/v |
| Ethylan TU (a conyl phenol/ethylene oxide condensate containing 10 moles of ethylene oxide per mol of phenol) | 10% w/v |
| Bentone 38 (an organic derivative of special magnesium montmorillonite thickener) | 0.5% w/v |
| Aromasol H (an aromatic solvent consisting predominantly of isomeric trimethylbenzenes) | to 100% by volume |

by intimately mixing the ingredients and grinding in a ball-mill for 24 hours. The emulsifiable suspension concentrate thus obtained may be diluted with water and applied at an application rate of 2.0 kg of emulsifiable suspension concentrate in 100 litres of spray fluid per hectare to control the growth of *Digitaria sanguinalis, Amaranthus retroflexus* and *Sinapis arvensis* by pre-emergence application in a crop of soya beans before emergence of the crop and weeds.

Similar emulsifiable suspension concentrates may be prepared as described above by replacing the N-(4-fluorophenyl)-2-(3-trifluoromethylphenoxy)nicotinamide by other compounds of general formula I.

Example 6

Granules were formed from:—

| | |
|---|---|
| N-(4-fluorophenyl)-2-(3-trifluoromethylphenoxy) nicotinamide | 5% w/w |
| Ethylan BCP | 1% w/w |
| Oleic acid | 1% w/w |
| Aromasol H | 12% w/w |
| 30/60 Attapulgite granules (sorptive silica clay) | 81% w/w |

by mixing the nicotinamide derivative, Ethylan BCP, oleic acid and Aromasol H and spraying the mixture onto the Attapulgite granules. The granules thus obtained may be applied at an application rate of 20 kg of granules per hectare to control the growth of *Echinochloa crusgalli, Eleocharis acicularis* and *Monochoria vaginalis* by pre-emergence application or application to seedling weeds in a crop of transplanted paddy rice.

Similar granules may be prepared as described above by replacing the N-(4-fluorophenyl)-2-(3-trifluoromethylphenoxy)nicotinamide by other compounds of general formula I.

Example 7

A water soluble concentrate was formed from:—

| | |
|---|---|
| N-(4-fluorophenyl)-2-(3-trifluoromethylphenoxy)-nicotinamide | 10% w/v |
| Ethylan KEO | 10% w/v |
| Dimethylformamide | to 100% by volume |

by dissolving the Ethylan KEO in a portion of dimethylformamide and then adding the nicotinamide derivative with heating and stirring until dissolved. The resulting solution was then made up to 100% by volume with dimethylformamide by adding the rest of the dimethylformamide. The water soluble concentrate thus obtained may be diluted with water and applied at an application rate of 10 litres of water soluble concentrate in 200 to 2000 litres of spray fluid per hectare to control the growth of *Galium aparine, Veronica persica,* and *Stellaria media* by pre-emergence application in a crop of winter wheat before emergence of the crop and weeds.

Example 8

A wettable powder was formed from:—

| | |
|---|---|
| N-(4-fluorophenyl)-2-(3-trifluoromethylphenoxy) nicotinamide | 90% w/w |
| Arylan S (sodium dodecyl benzene sulphonate) | 2% w/w |
| Darvan No. 2 (sodium lignosulphate) | 5% w/w |
| Celite PF | 3% w/w |

by mixing the ingredients and grinding the mixture in a hammer-mill to give a wettable powder which may be diluted with water and applied at an application rate of 1.0 kg of wettable powder in 300 litres of spray fluid per hectare to control the growth of *Galium aparine, Veronica persica* and *Stellaria media* by post-emergence application in an emerged crop of winter wheat.

Similar wettable powders may be prepared as described above by replacing the N-(4-fluorophenyl)-2-(3-trifluoromethylphenoxy)nicotinamide by other compounds of general formula I.

Example 9

A wettable powder containing 50% w/w of N-(4-fluorophenyl)-2-(3-trifluoromethylphenoxy)nicotinamide prepared as hereinbefore described in Example 2, may be diluted with water and applied at an application rate of 0.2 kg of wettable powder in 300 litres of spray fluid per hectare to control the growth of *Veronica persica* by pre-emergence application in a crop of winter wheat before emergence of the crop and weeds.

Example 10

A wettable powder containing 50% w/w of N-(4-fluorophenyl)-2-(3-trifluoromethylphenoxy)-nicotinamide prepared as described in Example 2, may be diluted with water and applied at an application rate of 40 kg of wettable powder in 600 litres of spray fluid per hectare to produce a total herbicidal effect on vegetation at a locus which is not a crop-growing area.

Example 11

An emulsifiable concentrate was formed from:—

| | |
|---|---|
| N-(4-fluorophenyl)-2-(3-trifluoromethylphenoxy) nicotinamide | 10% w/v |
| Soprophor BSU (condensate of tristyrylphenol and ethylene oxide, containing 18 moles of ethylene oxide) | 3.75% w/v |
| Arylan CA (70% solution of calcium dodecyl benzene sulphonate) | 3.75% w/v |
| Isophorone | 60% w/v |
| Aromasol H | to 100% by volume, |

by dissolving the Soprophor BSU and Arylan CA in a portion of the isophorone and then adding the nicotinamide derivative, with heating, and stirring until dissolved. The remaining isophorone was then added and the solution was made up to 100% by volume by adding the Aromasol H. The emulsifiable concentrate thus obtained may be diluted with water and applied at an application rate of 5 litre of emulsifiable concentrate in 200 litres of spray fluid per hextare to control the growth of *Galium aparine, Stellaria media, Veronica persica* and *Polygonum aviculare* by post-emergence application in an emerged crop of winter wheat.

Similar emulsifiable concentrates may be prepared as described above by replacing the N-(4-fluorophenyl)-2-(3-trifluoromethylphenoxy)nicotinamide by other compounds of general formula I.

In experiments on herbicidal activity carried out on representative compounds of general formula I, the following results have been obtained:—

TEST METHOD
Weed Control Test

(a) General

Appropriate quantities of the test compounds, hereinafter identified by the Compound Nos. hereinbefore indicated, were dissolved in acetone to give solutions equivalent to application rates of 0.25, 0.5, 1, 2, 4 or 8 kg of test compound per hectare. These solutions were applied from a standard laboratory herbicide sprayer using a flat fan jet travelling at 1.6 m.p.h (2.6 km/hour) and delivering the equivalent of 530 litres of spray fluid per hectare.

(b) Weed Control: Pre-emergence application

Weed seeds were sown on the surface of John Innes No. 1 potting compost (7 parts by volume of sterilized loam, 3 parts by volume of peat and 2 parts by volume of fine grit) in 9 cm diameter bitumenised paper pots. The quantities of seed per pot were as follows:—

| | Weed species | Approximate number seeds/pot |
|---|---|---|
| (i) | Broad leafed weeds | |
| | *Sinapis arvensis* | 30—40 |
| | *Polygonum lapathifolium* | 30—40 |
| | *Stellaria media* | 30—40 |
| (ii) | Grass weeds | |
| | *Avena fatua* | 15—20 |
| | *Alopecurus myosuroides* | 30—40 |
| | *Echinochloa crus-galli* | 20—30 |

The test compounds were applied to the uncovered seeds as described in (a) above at dose rates equivalent to 0.25, 0.5, 1, 2, 4 or 8 kg of test compound per hectare within the dose ranges hereinafter indicated and the seeds were covered with 25 ml of sharp sand after spraying. A single pot of each weed species was allocated to each treatment, with unsprayed controls and controls sprayed with acetone alone. After treatment, the pots were kept in the greenhouse and were watered overhead. Visual assessment of weed control activity was made 19 to 28 days after spraying. The results were expressed as the minimum

effective dose (MED) in kg/ha which gave 90% reduction in growth or kill of the weeds in comparison with plants in the control pots. The results obtained are presented below in Table I.

(c) Weed Control: Post-emergence application

Weed species were grown and then transplanted at the seedling state into John Innes No. 1 potting compost in 9 cm diameter bitumenised paper pots, except for *Avena fatua*, which was sown directly in the test pot and not transplanted. The plants were then grown in the greenhouse until ready for spraying with the test compounds. The number of plants per pot and the growth of the plant at spraying were as follows:—

| | Weed species | Number of plants/pot | Growth stages at spraying |
|---|---|---|---|
| (i) | Broad leafed weeds | | |
| | *Polygonum lapathifolium* | 5 | 1—1½ pairs of leaves |
| | *Stellaria media* | 5 | 4—6 leaves |
| | *Abutilon theophrasti* | 3 | 2 pairs of leaves |
| (ii) | Grass Weeds | | |
| | *Avena fatua* | 10 | 1 leaf |
| | *Alopecurus myosuroides* | 5 | 1½ leaves |
| | *Echinochloa crus-galli* | 5 | 1—2 leaves |

The test compounds were applied to the plants as described in (1) (a) above at dose rates equivalent to 0.25, 0.5, 1, 2, 4 and 8 kg of test compound per hectare within the dose ranges hereinafter indicated. A single pot of each weed species was allocated to each treatment, with unsprayed controls and controls sprayed with acetone alone. After spraying, the pots were watered overhead, commencing 24 hours after spraying. Assessment of the control of the growth of the weeds were made 19—28 days after spraying by recording the number of plants which had been killed and the reduction in growth. The results were expressed as the minimum effective dose (MED) in kg/ha which gave 90% reduction in growth or kill of the weeds in comparison with the plants in the control pots. The results obtained are presented below in Table II.

KEY TO WEED SPECIES

(a) *GRASS WEEDS:—*

Am = *Alopecurus myosuroides*

Af = *Avena fatua*

Ec = *Echinochloa crus-galli*

(b) *BROAD-LEAF WEEDS:—*

Sm = *Stellaria media*

Pl = *Polygonum lapathifolium*

Sa = *Sinapis arvensis*

At = *Abutilon theophrasti*

TABLE I

| Compound No. | PRE-EMERGENCE MED (kg/ha) | | | | | | Dose Range (kg/ha) |
|---|---|---|---|---|---|---|---|
| | Pl | Sa | Sm | Am | Af | Ec | |
| 1 | 0.5–1 | <0.25 | 0.25 | <0.25 | 0.5–1 | 0.5 | 0.25–2 |
| 2 | >4 | <1 | <1 | <1 | >4 | <1 | 1–4 |
| 3 | <1 | <1 | <1 | <1 | <1 | <1 | 1–4 |
| 4 | 2 | <1 | <1 | <1 | 2 | 2–4 | 1–4 |
| 5 | >>4 | <1 | <1 | <1 | >>4 | 1 | 1–4 |
| 7 | >4 | <0.5 | <0.5 | 0.5 | >4 | >4 | 0.5–4 |
| 9 | 2 | <1 | <1 | <1 | 1 | <1 | 1–4 |
| 12 | 4 | <0.5 | <0.5 | <0.5 | <0.5 | <0.5 | 0.5–4 |
| 13 | <0.5 | <0.5 | <0.5 | 1–2 | 1 | 2 | 0.5–4 |
| 15 | >2 | 0.25–0.5 | 0.25–0.5 | 0.5–1 | >2 | 1 | 0.25–2 |
| 16 | 2–4 | <2 | <2 | <2 | <2 | <2 | 2–8 |
| 17 | NR | <0.5 | >2 | 0.5 | >2 | <0.5 | 0.5–2 |
| 18 | NR | 2 | >>8 | >>8 | >>8 | >8 | 2–8 |
| 19 | 1 | <0.5 | <0.5 | 0.5–1 | >2 | 0.5 | 0.5–2 |
| 20 | 2 | 0.5–1 | <0.5 | 1 | >2 | 0.5–1 | 0.5–2 |
| 21 | 0.5–1 | <0.5 | <0.5 | <0.5 | 1–2 | 0.5 | 0.5–2 |
| 22 | 1 | <0.5 | <0.5 | 1 | 1–2 | 0.5–1 | 0.5–2 |
| 23 | 2 | <0.5 | 0.5 | 0.5 | 2–4 | 1 | 0.5–4 |
| 24 | >2 | <0.5 | <0.5 | 1–2 | 1 | 0.5 | 0.5–4 |
| 25 | >4 | 0.5–1 | 0.5–1 | >>4 | >>4 | >>4 | 0.5–4 |
| 26 | >4 | <1 | <1 | 1 | 2 | 2 | 1–4 |
| 27 | 2 | <0.5 | <0.5 | <0.5 | <0.5 | 1 | 0.5–4 |
| 28 | NR | <0.25 | 0.5–1 | 0.25 | >2 | >2 | 0.25–2 |
| 29 | NR | <0.5 | 1–2 | 0.5 | >>4 | 2 | 0.5–4 |
| 30 | >4 | <0.5 | <0.5 | 2–4 | >4 | >4 | 0.5–4 |

TABLE I (Continued)

| Compound No. | PRE-EMERGENCE MED (kg/ha) | | | | | | Dose Range (kg/ha) |
|---|---|---|---|---|---|---|---|
| | Pl | Sa | Sm | Am | Af | Ec | |
| 31 | >>2 | <0.5 | <0.5 | 1.0 | >>2 | 1–2 | 0.5–2 |
| 32 | NR | <0.5 | <0.5 | <0.5 | >>2 | 1–2 | 0.5–2 |
| 33 | NR | <2 | 2 | >8 | NR | >8 | 2–8 |
| 34 | NR | 0.5–1 | 1 | 0.5–1 | >>2 | 2 | 0.5–2 |
| 35 | NR | >8 | <2 | <2 | NR | <2 | 2–8 |
| 36 | NR | 4–8 | NR | >>8 | NR | NR | 2–8 |
| 38 | 0.5–1 | <0.5 | <0.5 | 1–2 | >2 | 1 | 0.5–2 |
| 39 | >4 | <0.5 | 0.5 | <0.5 | 4 | 0.5 | 0.5–4 |
| 41 | 2–4 | <1 | <1 | 2–4 | >4 | >4 | 1–4 |
| 42 | >>8 | <2 | <2 | 2–4 | 8 | 2–4 | 2–8 |
| 43 | NR | 1–2 | 2–4 | 4 | >4 | >4 | 1–8 |
| 44 | 2 | <0.5 | 0.5 | 0.5–1 | >2 | 0.5–1 | 0.5–2 |
| 45 | NR | NR | 4–8 | NR | NR | NR | 2–8 |
| 46 | >>8 | 2–4 | 2 | >8 | NR | >8 | 2–8 |
| 47 | NR | NR | <2 | >>8 | >>8 | >8 | 2–8 |
| 48 | >>8 | <2 | 2 | 8 | >>8 | >8 | 2–8 |
| 49 | >2 | <0.5 | 0.5–1 | 0.5–1 | 2 | 1 | 0.5–2 |
| 50 | 1–2 | <0.5 | <0.5 | 0.5 | 0.5–1 | <0.5 | 0.5–4 |
| 51 | 4 | <0.5 | <0.5 | <0.5 | 0.5–1 | <0.5 | 0.5–4 |
| 52 | >>2 | 0.25 | 0.5–1 | 0.25 | >2 | 2 | 0.25–2 |
| 53 | 2 | <0.5 | <0.5 | 2 | 2–4 | 2 | 0.5–4 |
| 55 | >4 | <0.5 | <0.5 | 0.5 | 2 | 4 | 0.5–4 |
| 56 | 0.5 | <0.5 | <0.5 | <0.5 | <0.5 | <0.5 | 0.5–4 |

TABLE II

| Compound No. | POST-EMERGENCE MED (kg/ha) | | | | | | Dose Range (kg/ha) |
|---|---|---|---|---|---|---|---|
| | At | Pl | Sm | Am | Af | Ec | |
| 1 | 0.5 | 1 | <0.25 | NR | >>2 | >>2 | 0.25–2 |
| 2 | <1 | <1 | 4 | NR | NR | 4 | 1–4 |
| 3 | <1 | 1 | <1 | >>4 | >4 | 4 | 1–4 |
| 4 | <1 | <1 | <1 | >>4 | >>4 | >>4 | 1–4 |
| 5 | <1 | >4 | 4 | >>4 | NR | >4 | 1–4 |
| 7 | 1 | 4 | 2 | >4 | >4 | >4 | 0.5–4 |
| 9 | <1 | 1 | 1 | >>4 | >>4 | >4 | 1–4 |
| 12 | <0.5 | 4 | 0.5 | >>4 | 4 | >4 | 0.5–4 |
| 13 | 2–4 | 4 | 2 | NR | NR | >8 | 0.5–4 |
| 15 | 4–8 | >8 | 4 | >>8 | 8 | >>8 | 2–8 |
| 16 | <2 | <2 | <2 | >8 | >>8 | >8 | 2–8 |
| 17 | NR | NR | NR | NR | NR | NR | 0.5–2 |
| 18 | >>8 | >8 | 4–8 | NR | NR | NR | 2–8 |
| 19 | 0.5–1 | 1 | 0.5–1 | >>2 | NR | >2 | 0.5–2 |
| 20 | 8 | NR | >8 | NR | >>8 | >>8 | 2–8 |
| 21 | 0.5 | 1 | 2 | >>2 | >>2 | >>2 | 0.5–2 |
| 22 | 4 | NR | >8 | NR | >8 | >8 | 2–8 |
| 23 | 0.5–1 | >4 | 4 | >>4 | >>4 | >>4 | 0.5–4 |
| 24 | 2 | 1–2 | 4 | NR | >4 | NR | 0.5–4 |
| 25 | 4 | >>4 | >>4 | NR | >>4 | NR | 0.5–4 |
| 26 | >>4 | 4 | NR | >>4 | NR | NR | 1–4 |
| 27 | 2 | >4 | >4 | NR | NR | NR | 0.5–4 |
| 28 | 2 | >2 | 2 | – | >>2 | >>2 | 0.25–2 |
| 29 | >4 | >>4 | >>4 | NR | >>4 | NR | 0.5–4 |
| 30 | 4 | NR | 4 | NR | NR | NR | 0.5–4 |

TABLE II (Continued)

| Compound No. | POST-EMERGENCE MED (kg/ha) | | | | | | Dose Range (kg/ha) |
|---|---|---|---|---|---|---|---|
| | At | Pl | Sm | Am | Af | Ec | |
| 31 | >8 | >>8 | 8 | >8 | >>8 | >>8 | 0.5–8 |
| 32 | 0.5–1 | >2 | >2 | NR | NR | NR | 0.5–2 |
| 33 | 4–8 | >>8 | >>8 | NR | >>8 | NR | 2–8 |
| 34 | 4–8 | >8 | 4–8 | >>8 | NR | NR | 2–8 |
| 35 | 2 –4 | >>8 | >8 | NR | NR | NR | 2–8 |
| 36 | >>8 | >>8 | >>8 | NR | NR | NR | 2–8 |
| 38 | 1 | 2 | 1–2 | >>2 | NR | NR | 0.5–2 |
| 39 | >4 | >>4 | >>4 | >>4 | NR | NR | 0.5–4 |
| 41 | 4 | >4 | 4 | NR | NR | NR | 1–4 |
| 42 | 2 | >>8 | >>8 | NR | NR | NR | 2–8 |
| 43 | 2 | 4 | 4–8 | >>8 | NR | >>8 | 1–8 |
| 44 | >>8 | >>8 | NR | NR | NR | NR | 0.5–8 |
| 45 | NR | NR | NR | NR | NR | NR | 2–8 |
| 46 | NR | NR | NR | NR | NR | NR | 2–8 |
| 47 | NR | NR | NR | NR | NR | NR | 2–8 |
| 48 | >>8 | >>8 | >>8 | NR | NR | NR | 2–8 |
| 49 | <2 | <2 | 4 | >>8 | >>8 | >8 | 2–8 |
| 50 | 0.5 | <0.5 | <0.5 | >>4 | >>4 | 0.5 | 0.5–4 |
| 51 | 0.5–1 | 2 | <0.5 | >4 | >4 | >4 | 0.5–4 |
| 52 | 2 | >>2 | 2 | – | NR | NR | 0.25–2 |
| 53 | 0.5 | >4 | 2 | NR | >4 | >>4 | 0.5–4 |
| 55 | 0.5–1 | >>4 | 1–2 | NR | >>4 | >>4 | 0.5–4 |
| 56 | 0.5 | >>4 | 0.5 | >4 | 2–4 | >>4 | 0.5–4 |

The following symbols which appear in the above Tables have the following meanings:—

'>>' means = much greater than

'>' means = greater than

'<' means = less than

'NR' means = no reduction at any dose rate applied

'—' means = not tested

The compounds of general formula I may be prepared by the application or adaptation of known methods for the preparation of nicotinamide derivatives.

According to a feature of the present invention, the new nicotinamide derivatives of general formula I, wherein X represents an oxygen atom and $R^1$, $R^2$, $R^3$, $R^4$, $R^5$, $R^6$, $R^7$, $R^8$ and *m* are as hereinbefore defined, are prepared by the process which comprises the reaction of a compound of the general formula:—

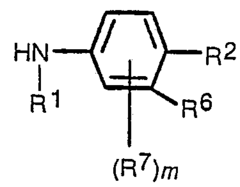

(II)

(wherein $R^1$, $R^2$, $R^6$, $R^7$ and *m* are as hereinbefore defined), or an acid addition salt thereof, e.g. the hydrochloride, with a compound of the general formula:—

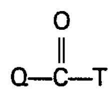

(III)

wherein Q represents a group of the general formula:—

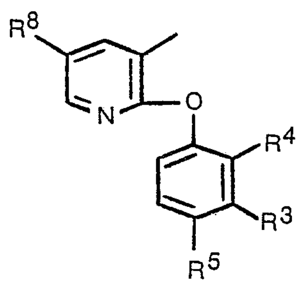

(IV)

(wherein $R^3$, $R^4$, $R^5$ and $R^8$ are as hereinbefore defined) and T represents a bromine or, preferably, chlorine atom or a group of the general formula:—

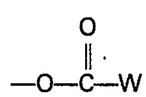

(V)

wherein W represents a group of general formula IV (wherein $R^3$, $R^4$, $R^5$ and $R^8$ are as hereinbefore defined) or a straight- or branched-chain alkyl group containing from 1 to 4 carbon atoms.

The reaction between the compounds of general formula II, or acid addition salts thereof, and the compounds of general formula III may be effected in the presence of a suitable inert organic solvent, for example an aromatic hydrocarbon e.g. benzene or toluene, or dimethylformamide or a halohydrocarbon, e.g. dichloromethane or tetrachloroethane, and at a temperature of from the ambient temperature to the reflux temperature of the reaction mixture and optionally in the presence of a base, for example triethylamine or potassium carbonate.

According to a further feature of the present invention, the new nicotinamide derivatives of general formula I, wherein X represents an oxygen atom and $R^1$, $R^2$, $R^3$, $R^4$, $R^5$, $R^6$, $R^7$, $R^8$ and *m* are as hereinbefore defined, are prepared by the process which comprises the reaction of a compound of the general formula:—

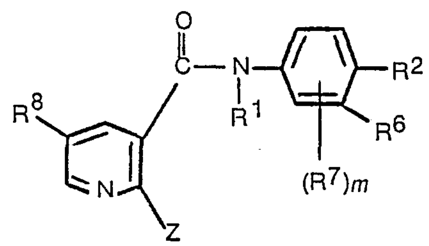

(VI)

(wherein Z represents a chlorine or bromine atom and $R^1$, $R^2$, $R^6$, $R^7$, $R^8$ and *m* are as hereinbefore defined) with an alkali metal, e.g. sodium or potassium, salt of a compound of the general formula:—

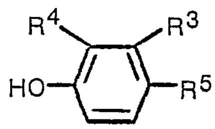

(VII)

wherein $R^3$, $R^4$ and $R^5$ are as hereinbefore defined.

The reaction between the compound of general formula VI and the sodium salts of the compounds of general formula VII may be effected by heating the compound of general formula VI with the sodium salt of the compound of general formula VII in a suitable inert organic solvent, for example diethyleneglycol dimethyl ether, or preferably, in the presence of the compound of general formula VII, which serves as a solvent medium for the reaction, or by heating the compound of general formula VI with a solution of sodium hydride and the compound of general formula VII in dimethylformamide. The reaction is preferably effected at a temperature of from 100°C to the reflux temperature of the reaction mixture.

The reaction of the compounds of general formula VI and the potassium salts of the compounds of general formula VII may be effected by heating a mixture of the compound of general formula VI, the compound of general formula VII and potassium carbonate in a suitable inert aprotic organic solvent, e.g. dimethylformamide or dimethylsulphoxide. The reaction is preferably effected at a temperature of from 100°C to the reflux temperature of the reaction mixture.

The compounds of general formula VI may be prepared by the reaction of a compound of general formula II, wherein the various symbols are as hereinbefore defined, or an acid addition salt thereof, with a compound of the general formula:—

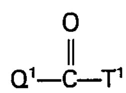

(VIII)

wherein $Q^1$ represents a group of the general formula:—

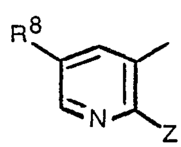

(IX)

(wherein $R^8$ and Z are as hereinbefore defined) and $T^1$ represents a bromine or, preferably, chlorine atom or a group of the general formula:—

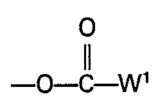

(X)

wherein $W^1$ represents a group of general formula IX (wherein $R^8$ and Z are as hereinbefore defined) or a straight- or branched-chain alkyl group containing from 1 to 4 carbon atoms.

The reaction between the compounds of general formula II, or acid addition salts thereof, and the compounds of general formula VIII may be effected as hereinbefore described for the reaction of compounds of general formula II, or acid addition salts thereof, with the compounds of general formula III.

The compounds of general formula III, wherein Q and T are as hereinbefore defined may be prepared from compounds of the general formula:—

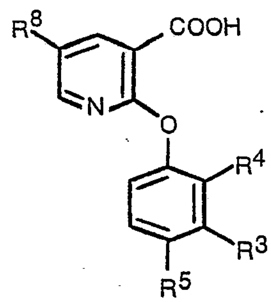

(XI)

wherein $R^3$, $R^4$, $R^5$ and $R^8$ are as hereinbefore defined, by known methods for the preparation of carboxylic acid chlorides, bromides, anhydrides or mixed anhydrides, for example by reaction of a compound of general formula XI with thionyl chloride or bromide, acetic anhydride or an alkylchloroformate, wherein the alkyl moiety contains from 1 to 4 carbon atoms, e.g. methyl chloroformate or ethyl chloroformate.

The compounds of general formula XI may be prepared according to the procedure described by P. J. Villani *et al*, J. Med. Chem. *18*, 1, (1975), for example by the reaction of 2-chloronicotinic acid with a compound of general formula VII, wherein the various symbols are as hereinbefore defined, in the presence of sodium metal and methanol.

The compounds of general formula VIII may be prepared from 2-chloronicotinic acids or 2-bromonicotinic acids by application of the procedures hereinbefore described for the preparation of compounds of general formula III from compounds of general formula XI.

According to a further feature of the present invention, the compounds of general formula I wherein X represents a sulphur atom and $R^1$, $R^2$, $R^3$, $R^4$, $R^5$, $R^6$, $R^7$, $R^8$ and *m* are as hereinbefore defined are prepared by the reaction of a compound of general formula I wherein X represents an oxygen atom and the other symbols are as hereinbefore defined, with phosphorus pentasulphide.

The reaction of compounds of general formula II wherein X represents an oxygen atom and the other symbols are as hereinbefore defined, with phosphorus pentasulphide may be effected in a suitable inert organic solvent, for example an aromatic hydrocarbon, e.g. benzene or toluene, pyridine or quinoline, and preferably with heating, e.g. at the reflux temperature of the reaction mixture.

The following Examples and Reference Examples illustrate the preparation of the compounds of the invention.

## Example 12

Compounds Numbers 1 to 5, 7, 9, 12, 53, 13, 15 to 30, 55, 56, 31 to 36, 38, 39 and 41 to 48

Thionyl chloride (28.6 g) was added over 5 minutes to a stirred mixture of 2-(3-trifluoromethylphenoxy)nicotinic acid [68 g; described by F. J. Villani *et al*, J. Med. Chem. *18*, 1 (1975)] in toluene (400 ml). The mixture was heated on the steam bath for 18 hours and was cooled to 30°C and evaporated under reduced pressure. The residue was dissolved in toluene (400 ml) and treated with stirring with triethylamine (48.6 g) at ambient temperature. 4-Fluoroaniline (26.2 g) was added and the mixture was heated on the steam bath for 5 hours. The thick paste was evaporated under reduced pressure and the residue was triturated with water (250 ml).

The aqueous mixture was extracted with methylene chloride (2 × 250 ml) and the combined methylene chloride extracts were washed with water (150 ml), dried over sodium sulphate and evaporated under reduced pressure to give a brown solid which was recrystallised from a mixture of toluene and hexane (850 ml; 1:9) to give Compound (Cpd.) 1, N-(4-fluorophenyl)-2-(3-trifluoromethylphenoxy)-nicotinamide (78.2 g), m.p. 131—132°C, as off-white crystals. By proceeding in a similar manner, but replacing the 4-fluoroaniline by the appropriately substituted anilines, there were prepared:—

(Cpd. 2) N-phenyl-2-(3-trifluoromethylphenoxy)nicotinamide, m.p. 164—5°C, (after crystallisation from toluene), from aniline:

(Cpd. 3) N-(2,4-difluorophenyl)-2-(3-trifluoromethylphenoxy)nicotinamide, m.p. 161—2°C, (after crystallisation from toluene), from 2,4-difluoroaniline:

(Cpd. 4) N-(3-chloro-4-fluorophenyl)-2-(3-trifluoromethylphenoxy)nicotinamide, m.p. 111—2°C, (after crystallisation from toluene), from 3-chloro-4-fluoroaniline:

(Cpd. 5) N-(4-methylphenyl)-2-(3-trifluoromethylphenoxy)nicotinamide, m.p. 162—3°C, (after crystallisation from toluene, from 4-methylaniline:

(Cpd. 7) N-(2,4,6-trifluorophenyl)-2-(3-trifluoromethylphenoxy)nicotinamide, m.p. 168—9°C, (after crystallisation from a mixture of hexane and toluene) from 2,4,6-trifluoroaniline:

(Cpd. 9) N-(3,4-difluorophenyl)-2-(3-trifluoromethylphenoxy)nicotinamide, m.p. 150—1°C, (after crystallisation from toluene), from 3,4-difluoroaniline:

(Cpd. 12) N-(2,4,5-trifluorophenyl)-2-(3-trifluoromethylphenoxy)nicotinamide, m.p. 164—5°C, (after crystallisation from a mixture of hexane and toluene), from 2,4,5-trifluoroaniline:

(Cpd. 53) N-methyl-N-phenyl-2-(3-trifluoromethylphenoxy)nicotinamide, as a clear mobile oil, (refractive index $N_D^{20}$ = 1,5612) from N-methylaniline:

(Cpd. 13) N-(4-fluorophenyl)-N-methyl-2-(3-trifluoromethylphenoxy)nicotinamide, m.p. 109—110°C, (after crystallisation from hexane) from 4-fluoro-N-methylaniline [described by R. Cervellati *et al*, J. Mol. Struct., *56*, 69 (1979)]:

By proceeding in a similar manner, but replacing the 2-(3-trifluoromethylphenoxy)nicotinic acid by the appropriately substituted phenoxy-nicotinic acid, there were prepared:—

(Cpd. 15) 2-(3-fluorophenoxy)-N-(4-fluorophenyl)nicotinamide, m.p. 162.5—163°C, (after crystallisation from toluene), from 2-(3-fluorophenoxy)nicotinic acid [described by F. J. Villani *et al*, J. Med. Chem *18*, 1 (1975)]:

(Cpd. 16) 2-(3-chlorophenoxy)-N-(4-fluorophenyl)nicotinamide, m.p. 161—162°C, (after crystallisation from toluene), from 2-(3-chlorophenoxy)nicotinic acid [described by F. J. Villani et al, J. Med. Chem *18*, 1 (1975)]:

(Cpd. 17) 2-(2,3-dichlorophenoxy)-N-(4-fluorophenyl)nicotinamide, m.p. 171—172°C, (after crystallisation from ethanol), from 2-(2,3-dichlorophenoxy)nicotinic acid:

(Cpd. 18) 2-(3,4-dichlorophenoxy)-N-(4-fluorophenyl) nicotinamide, m.p. 122—123°C, (after crystallisation from toluene), from 2-(3,4-dichlorophenoxy)nicotinic acid:

(Cpd. 19) 2-(3-bromophenoxy)-N-(4-fluorophenyl)nicotinamide, m.p. 147.5—148.5°C, (after crystallisation from toluene), from 2-(3-bromophenoxy)nicotinic acid:

(Cpd. 20) N-(4-fluorophenyl)-2-(3-methoxyphenoxy)nicotinamide, m.p. 138—140°C, (after crystallisation from toluene), from 2-(3-methoxyphenoxy)nicotinic acid [described by F. J. Villani *et al*, J. Med. Chem. *18*, 1 (1975)]:

(Cpd. 21) 2-(3-cyanophenoxy)-N-(4-fluorophenyl)nicotinamide, m.p. 167—168°C, (after crystallisation from ethanol), from 2-(3-cyanophenoxy)nicotinic acid:

(Cpd. 22) N-(4-fluorophenyl)-2-(3-methanesulphonylphenoxy)nicotinamide, m.p. 180—182°C, (after crystallisation from ethanol), from 2-(3-methanesulphonylphenoxy)nicotinic acid:

(Cpd. 23) 2-(3-chloro-4-fluorophenoxy)-N-(4-fluorophenyl)nicotinamide, m.p. 115—7°C, (after crystallisation from a mixture of hexane and toluene), from 2-(3-chloro-4-fluorophenoxy)nicotinic acid:

(Cpd. 24) 2-(4-chloro-3-trifluoromethylphenoxy)-N-(4-fluorophenyl)nicotinamide, m.p. 128—9°C, (after crystallisation from hexane), from 2-(4-chloro-3-trifluoromethylphenoxy)nicotinic acid:

(Cpd. 25) 2-(3-ethanesulphonylphenoxy)-N-(4-fluorophenyl)nicotinamide, m.p. 168—9°C (after crystallisation from a mixture of hexane and toluene), from 2-(3-ethanesulphonylphenoxy)nicotinic acid.

(Cpd. 26) 2-(3,4-difluorophenoxy)-N-(4-fluorophenyl)nicotinamide, m.p. 137—139°C, (after crystallisation from a mixture of hexane and toluene), from 2-(3,4-difluorophenoxy)nicotinic acid:

By proceeding in a similar manner but replacing the 4-fluoroaniline by 2,4-difluoroaniline and replacing the 2-(3-trifluoromethylphenoxy)nicotinic acid by the appropriately substituted phenoxynicotinic acid, there were prepared the following compounds:—

(Cpd. 27) N-(2,4-difluorophenyl)-2-(3-methanesulphonylphenoxy)nicotinamide, m.p. 197—8°C, (after crystallisation from a mixture of hexane and toluene), from 2-(3-methanesulphonylphenoxy)nicotinic acid:

(Cpd. 28) 2-(3-bromophenoxy)-N-(2,4-difluorophenyl)nicotinamide, m.p 178—9°C, (after crystallisation from a mixture of hexane and toluene), from 2-(3-bromophenoxy)nicotinic acid:

(Cpd. 29) 2-(3-chloro-4-fluorophenoxy)-N-(2,4-difluorophenyl)nicotinamide, m.p. 172—3°C, (after crystallisation from a mixture of hexane and toluene), from 2-(3-chloro-4-fluorophenoxy)nicotinic acid:

(Cpd. 30) 2-(3-ethanesulphonylphenoxy)-N-(2,4-difluorophenyl)nicotinamide, m.p. 170—1°C, (after crystallisation from a mixture of hexane and toluene), from 2-(3-ethanesulphonylphenoxy)nicotinic acid:

(Cpd. 55) 2-(4-chloro-3-trifluoromethylphenoxy)-N-(2,4-difluorophenyl)nicotinamide, m.p. 137—9°C, (after crystallisation from a mixture of hexane and toluene), from 2-(4-chloro-3-trifluoromethylphenoxy)-nicotinic acid:

(Cpd. 56) 2-(3-trifluoromethylphenoxy)-N-(2,4-difluorophenyl)-5-methylnicotinamide, m.p. 133—5°C, (after crystallisation from a mixture of hexane and toluene), from 2-(3-trifluoromethylphenoxy)-5-methyl-nicotinic acid.

By proceeding in a similar manner but replacing the 2-(3-trifluoromethylphenoxy)nicotinic acid by 2-(3-chlorophenoxy)nicotinic acid [described by F. J. Villani *et al*, J. Med. Chem. *18*, 1 (1975)] and replacing the 4-fluoroaniline by the appropriately substituted aniline, there were prepared the following compounds:—

(Cpd. 31) 2-(3-chlorophenoxy)-N-phenylnicotinamide, m.p. 156—158°C, (after crystallisation from ethanol), from aniline:

(Cpd. 32) 2-(3-chlorophenoxy)-N-(2,4-difluorophenyl)nicotinamide, m.p. 185—187°C, (after crystallisation from toluene), from 2,4-difluoroaniline:

(Cpd. 33) 2-(3-chlorophenoxy)-N-(2,4,6-trifluorophenyl)nicotinamide, m.p. 197—199°C, (after crystallisation from toluene), from 2,4,6-trifluoroaniline:

(Cpd. 34) N-(3-chloro-4-fluorophenyl)-2-(3-chlorophenoxy)nicotinamide, m.p. 160—162°C, (after crystallisation from toluene), from 3-chloro-4-fluoroaniline:

(Cpd. 35) 2-(3-chlorophenoxy)-N-(4-methylphenyl)nicotinamide, 172—174°C, (after crystallisation from toluene), from 4-methylaniline:

(Cpd. 36) 2-(3-chlorophenoxy)-N-(4-cyanophenyl)nicotinamide, m.p. 166—168°C, (after crystallisation from ethanol), from 4-aminobenzonitrile:

(Cpd. 38) 2-(3-chlorophenoxy)-N-(4-fluorophenyl)-N-methylnicotinamide, as a clear mobile oil, (refractive index $n_D^{20}$ = 1.6007) from 4-fluoro-N-methylaniline:

(Cpd. 39) 2-(3-chlorophenoxy-N-(2,4,5-trifluorophenyl)nicotinamide, m.p. 168—170°C, (after crystallisation from a mixture of hexane and toluene), from 2,4,5-trifluoroaniline:

(Cpd. 41) 2-(3-chlorophenoxy)-N-ethyl-N-(4-fluorophenyl)nicotinamide, m.p. 78—80°C, (after crystallisation from a mixture of diethylether and hexane) from N-ethyl-4-fluoro-aniline [described by F. L. Allen *et al*, J. Amer. Chem. Soc. 5259 (1960)]:

(Cpd. 42) 2-(3-chlorophenoxy)-N-(4-trifluoromethylphenyl) nicotinamide, m.p. 137—9°C, (after crystallisation from a mixture of hexane and toluene), from 4-trifluoromethylaniline:

(Cpd. 43) 2-(3-chlorophenoxy)-N-(3,4-difluorophenyl) nicotinamide, m.p. 171—3°C, (after crystallisation from a mixture of hexane and toluene), from 3,4-difluoroaniline:

By proceeding in a similar manner but replacing the 2-(3-trifluoromethylphenoxy)nicotinic acid by 2-(3-methoxyphenoxy)nicotinic acid and replacing the 4-fluoroaniline by the appropriately substituted anilines, there were prepared the following compounds:—

(Cpd. 44) 2-(3-methoxyphenoxy)-N-phenylnicotinamide, m.p. 129—131°C, (after crystallisation from a mixture of ethanol and water), from aniline:

(Cpd. 45) N-(2,4-difluorophenyl)-2-(3-methoxyphenoxy)nicotinamide, m.p. 175—176°C, (after crystallisation from a mixture of ethanol and water), from a 2,4-difluoroaniline:

(Cpd. 46) 2-(3-methoxyphenoxy)-N-(2,4,6-trifluorophenyl) nicotinamide, m.p. 133—135°C, (after crystallisation from a mixture of ethanol and water), from 2,4,6-trifluoroaniline:

(Cpd. 47) 2-(3-methoxyphenoxy)-N-(4-methylphenyl) nocotinamide, m.p. 159—160°C, (after crystallisation from a mixture of ethanol and water), from 4-methylaniline:

(Cpd. 48) N-(3-chloro-4-fluorophenyl)-2-(3-methoxyphenoxy) nicotinamide, m.p. 131—132°C, (after crystallisation from a mixture of ethanol and water), from 3-chloro-4-fluoroaniline:

## Example 13

Compounds Numbers 49, 50 and 51

A mixture of 2-(3-chlorophenoxy)-N-(4-fluorophenyl)nicotinamide (prepared as described in Example 12; 6.68 g), phosphorus pentasulphide (4 g) and anhydrous pyridine (20 ml) was heated at reflux for 4 hours, cooled and added to water (250 ml). The mixture thus obtained was extracted with methylene chloride (2 × 100 ml). The combined extracts were dried over sodium sulphate, evaporated to dryness under reduced pressure (20 mm Hg) and the solid residue was crystallised from a mixture of hexane and toluene (250 ml, 1:1) to give 2-(3-chlorophenoxy)-N-(4-fluorophenyl)thionicotinamide (4.1 g), m.p. 133—4°C, as a yellow solid (Cpd. 49).

By proceeding in a similar manner but replacing the 2-(3-chlorophenoxy)-N-(4-fluorophenyl)-nicotinamide by N-(4-fluorophenyl)-2-(3-trifluoromethylphenoxy)nicotinamide and N-(2,4-difluorophenyl)-2-(3-trifluoromethylphenoxy)nicotinamide (prepared as described in Example 12), there were prepared, respectively:—

(Cpd. 50) N-(4-fluorophenyl)-2-(3-trifluoromethylphenoxy) thionicotinamide, m.p. 128.5—129.5°C, (after crystallisation from hexane):

(Cpd. 51) N-(2,4-difluorophenyl)-2-(3-trifluoromethylphenoxy)thionicotinamide, m.p. 157—8°C, (after crystallisation from hexane).

## Example 14

Compound No. 52

2-Chloro-N-(2,4-difluorophenyl)nicotinamide (described in Belgian patent No. 827,567; 4.8 g) was added to a solution of 3-fluorophenol (2 g), sodium metal (0.4 g) and methanol (5 ml) in diethyleneglycol dimethyl ether (75 ml). Methanol was then removed by distillation. The reaction mixture was then heated at reflux for 18 hours, cooled and evaporated to dryness under reduced pressure (20 mm Hg). The solid residue was dissolved in methylene chloride (100 ml) and washed with 2N aqueous sodium hydroxide solution (2 × 150 ml) and water (4 × 100 ml). The methylene chloride solution was then dried over sodium sulphate and evaporated to dryness under reduced pressure (20 mm Hg). The solid residue thus obtained was crystallised from toluene (25 ml) to give N-(2,4-difluorophenyl)-2-(3-fluorophenoxy)-nicotinamide (1.9 g), m.p. 157—158.5°C, as a colourless crystalline solid.

## Example 15

Compound No. 16

2-Chloro-N-(4-fluorophenyl)nicotinamide (described in Belgian Patent No. 827,567; 5 g) was added to a solution of 3-chlorophenol (7.8 g) and sodium metal (0.5 g) in methanol (10 ml). Methanol was removed by distillation and the residue was heated, with stirring, at 180—190°C for 2 hours. After cooling, the reaction mixture was dissolved in methylene chloride (200 ml), and washed with 2N aqueous sodium hydroxide solution (2 × 150 ml) and water (3 × 150 ml). The methylene chloride solution was dried over sodium sulphate and evaporated to dryness under reduced pressure (20 mm Hg). The solid residue thus obtained was crystallised from toluene (50 ml) to give 2-(3-chlorophenoxy)-N-(4-fluorophenyl)nicotinamide (3.5 g), m.p. 161—2°C, as a colourless crystalline solid.

Reference Example 1

2-Chloronicotinic acid [50 g; described by P. Nantka-Namirski, Acta. Pol. Pharm. *23*, 403 (1966)] was added to a solution of 3-trifluoromethylphenol (250 g) and sodium metal (15 g) in methanol (150 ml). Methanol was removed by distillation and the residue was heated for 2 hours at 180—190°C. The cooled mixture was added to water (1500 ml) and the solution extracted with diethyl ether (3 × 250 ml). The aqueous layer was acidified with glacial acetic acid to precipitate a brown solid. The solid was collected on a filter, washed with water (250 ml) and recrystallised from isopropanol to give 2-(3-trifluoromethylphenoxy)nicotinic acid (68 g), m.p. 155—156°C, as off-white crystals. By proceeding in a similar manner but replacing the 3-trifluoromethylphenol by the appropriately substituted phenols, there were prepared the following intermediates:—

2-(2,3-dichlorophenoxy)nicotinic acid, m.p. 218—219°C, (after crystallisation from isopropanol), from 2,3-dichlorophenol:

2-(3-bromophenoxy)nicotinic acid, m.p. 169—170°C, (after crystallisation from isopropanol), from 3-bromophenol:

2-(3-cyanophenoxy)nicotinic acid, m.p. 230—231°C, (after crystallisation from isopropanol), from 3-cyanophenol:

2-(3-methanesulphonylphenoxy)nicotinic acid, m.p. 68—70°C, (after crystallisation from water), from 3-methanesulphonylphenol [described by T. Zincke and C. Ebel, Chem. Ber. *47*, 930 (1914)]:

2-(3-chloro-4-fluorophenoxy)nicotinic acid, m.p. 191—3°C, (after crystallisation from isopropanol), from 3-chloro-4-fluorophenol [described by G. C. Finger *et al*, J. Amer. Chem. Soc., *81*, 94 (1959)]:

2-(4-chloro-3-trifluoromethylphenoxy)nicotinic acid, m.p. 221—2°C, (after crystallisation from a mixture of isopropanol and water), from 4-chloro-3-trifluoromethylphenol [described by A. Mooradian *et al*, J. Amer. Chem. Soc., *73*, 3470 (1951)]:

2-(3,4-difluorophenoxy)nicotinic acid, m.p. 181—2°C, (after crystallisation from isopropanol), from 3,4-difluorophenol, [described by G. C. Finger *et al*, J. Amer. Chem. Soc. *81*, 94 (1959)]:

2-(3-ethanesulphonylphenoxy)nicotinic acid, m.p. 169—171°C, (after crystallisation from isopropanol), from 3-ethanesulphonylphenol.

By proceeding in a similar manner but replacing the 2-chloronicotinic by 2-bromo-5-methylnicotinic acid [described by J. J. Baldwin *et al*, J. Org. Chem. *43*, 2529 (1978)], there was prepared 2-(3-trifluoromethylphenoxy)-5-methylnicotinic acid, m.p. 173—5°C.

Reference Example 2

A mixture of methyl 2-(3,4-dichlorophenoxy) nicotinoate (8.3 g), sodium hydroxide (1.2 g), water (20 ml) and ethanol (27 ml) was heated at the reflux for 1 hour. The mixture was cooled and evaporated under reduced pressure. The residue was dissolved in water (200 ml) and the solution washed with diethyl ether (2 × 100 ml). The aqueous solution was acidified with glacial acetic acid to precipitate a solid. This was collected on a filter and washed with water (50 ml) to give 2-(3,4-dichlorophenoxy)nicotinic acid (6.8 g), m.p. 131—133°C, as a colourless solid.

Reference Example 3

Methyl 2-bromonicotinoate [26.8 g; described by T. A. Bryson *et al*, J. Org. Chem. *39*, 3436 (1974)] was added to a stirred mixture of potassium carbonate (25.7 g) and 3,4-dichlorophenol (20.3 g) in dimethylformamide (250 ml) and the mixture was heated for 18 hours at 125°C. The cooled solution was filtered and diluted with water (250 ml) to precipitate a solid. The solid was collected on a filter and recrystallised from hexane (30 ml) to give methyl 2-(3,4-dichlorophenoxy)nicotinoate (8.3 g), m.p. 94—5°C.

Reference Example 4

A stirred mixture of 3-ethanesulphonylaniline [24.8 g; described by G. D. Palmer and E. E. Reid, J. Chem. Soc. *48*, 528 (1926)] and concentrated sulphuric acid (46.2 ml) in water (74 ml) was treated at −5 to 0° with a solution of sodium nitrite (9.3 g) in water (23 ml). The solution thus obtained was then added to a boiling solution of concentrated sulphuric acid (92.4 ml) in water (64.7 ml) and when addition was complete, the mixture was heated at reflux for 5 minutes. After cooling, the reaction mixture was added to ice (500 g) and extracted with methylene chloride (3 × 150 ml). The combined methylene chloride extracts were dried over magnesium sulphate and evaporated under reduced pressure (20 mm Hg) to give 3-ethanesulphonylphenol (23.6 g) as a clear mobile oil.

4-Fluoroaniline, 2,4-difluoroaniline, 3-chloro-4-fluoroaniline, 2,4,6-trifluoroaniline, 3,4-difluoroaniline, 2,4,5-trifluoroaniline, 4-methylaniline, 4-aminobenzonitrile, 4-trifluoromethylaniline, N-methylaniline, 3-fluorophenol, 3-chlorophenol, 2-chloronicotinic acid, 3-trifluoromethylphenol, 2,3-dichlorophenol, 3-bromophenol, 3-cyanophenol, and 3,4-dichlorophenol used as starting materials in Examples 12, 14 and 15 and Reference Examples 1, 3 and 5 above, are known compounds which are readily available.

**Claims for the Contracting States: BE CH DE FR GB IT LI LU NL SE**

1. Nicotinamide derivatives of the general formula:

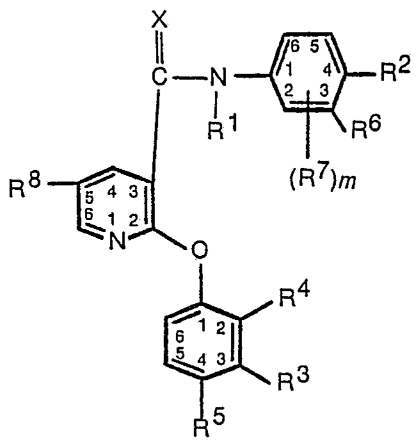

(I)

wherein X represents an oxygen or sulphur atom, $R^1$ represents a hydrogen atom or a methyl or ethyl group, $R^2$ represents a hydrogen or fluorine atom, a cyano group or a methyl or ethyl group optionally substituted by one or more fluorine atoms, which is preferably trifluoromethyl, $R^3$ represents a fluorine, chlorine or bromine atom or a cyano, trifluoromethyl, methoxy, ethoxy, trifluoromethoxy or alkanesulphonyl group, wherein the alkane moiety of the alkanesulphonyl group may be straight- or branched-chain and contains from 1 to 4 carbon atoms, one of $R^4$ and $R^5$ represents a hydrogen atom and the other represents a hydrogen, fluorine, chlorine or bromine atom or a cyano, trifluoromethyl, methoxy, ethoxy, trifluoromethoxy or alkanesulphonyl group, wherein the alkane moiety of the alkanesulphonyl group may be straight- or branched-chain and contains from 1 to 4 carbon atoms, $R^6$ represents a hydrogen, fluorine or chlorine atom, $R^7$ represents a fluorine a chlorine atom, $R^8$ represents a hydrogen, fluorine, chlorine or bromine atom or a methyl or ethyl group and *m* represents 0, 1 or 2, wherein, when *m* represents 2, the atoms represented by the symbol $R^7$ may be the same or different, with the provisos (1) that when $R^2$ represents a hydrogen atom, $R^6$ represents a hydrogen atom, *m* represents 0 and (2) that when $R^2$ represents a fluorine atom, a cyano group or a methyl or ethyl group optionally substituted by one or more fluorine atoms, and $R^6$ represents a fluorine or chlorine atom, *m* represents 0 or 1.

2. Nicotinamide derivatives according to claim 1, wherein $R^2$ represents a hydrogen or fluorine atom, a cyano group or a methyl or trifluoromethyl group, $R^3$ represents a fluorine, chlorine or bromine atom or a methoxy, cyano, trifluoromethyl, methanesulphonyl or ethanesulphonyl group, one of $R^4$ and $R^5$ represents a hydrogen atom and the other represents a hydrogen, fluorine or chlorine atom, $R^7$ represents a fluorine or chlorine atom, $R^8$ represents a hydrogen atom or a methyl group and X, $R^1$, $R^6$ and *m* are as defined in claim 1.

3. Nicotinamide derivatives according to claims 1 or 2, wherein X represents an oxygen or sulphur atom, $R^1$ represents a hydrogen atom or a methyl group, $R^2$ represents a hydrogen or fluorine atom, $R^3$ represents a trifluoromethyl group, $R^4$ and $R^5$ each represent a hydrogen atom, $R^6$ represents a hydrogen or fluorine atom, $R^7$ represents a fluorine atom, $R^8$ represents a hydrogen atom or a methyl group and *m* represents 0 or 1, with the provisos (i) that when $R^2$ represents a hydrogen atom, $R^6$ represents a hydrogen atom and *m* represents 0 and (ii) that when $R^2$ represents a fluorine atom, (a) $R^6$ represents a hydrogen atom and *m* represents 0 or *m* represents 1 and $R^7$ represents a fluorine atom in the 2- (or 6-) position of the phenyl ring, or (b) $R^6$ represents a fluorine atom and *m* represents 0, or *m* represents 1 and $R^7$ represents a fluorine atom in the 6-position of the phenyl ring.

4. N - (4 - Fluorophenyl) - 2 - (3 - trifluoromethylphenoxy) - nicotinamide, N - phenyl - 2 - (3 - trifluoromethylphenoxy)nicotinamide, N - (2,4 - difluorophenyl) - 2 - (3 - trifluoromethylphenoxy)-nicotinamide, N - (3,4 - difluorophenyl) - 2 - (3 - trifluoromethylphenoxy)nicotinamide, N - (2,4,5 - trifluorophenyl) - 2 - (3 - trifluoromethylphenoxy)nicotinamide, N - (4 - fluorophenyl) - N - methyl - 2 - (3 - trifluoromethylphenoxy)nicotinamide, N - (4 - fluorophenyl) - 2 - (3 - trifluoromethylphenoxy)thionicotinamide, 2 - (3 - trifluoromethylphenoxy) - N - (2,4 - difluorophenyl) - 5 - methylnicotinamide and N - methyl - N - phenyl - 2 - (3 - trifluoromethylphenoxy)nicotinamide.

5. A process for the preparation of nicotinamide derivatives of general formula I depicted in claim 1 which comprises:

(A) when X represents an oxygen atom and $R^1$, $R^2$, $R^3$, $R^4$, $R^5$, $R^6$, $R^7$, $R^8$ and *m* are as defined in claim 1, the reaction of a compound of the general formula:—

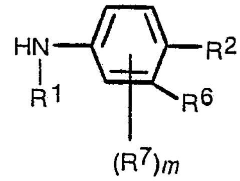

(II)

(wherein $R^1$, $R^2$, $R^6$, $R^7$ and *m* are as hereinbefore defined) or an acid addition salt thereof, with a compound of the general formula:—

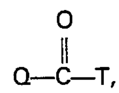

(III)

wherein Q represents a group of the general formula:—

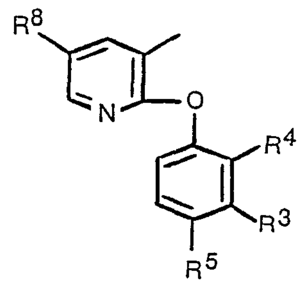

(IV)

(wherein $R^3$, $R^4$, $R^5$ and $R^8$ are as hereinbefore defined) and T represents a chlorine or bromine atom or a group of the general formula:—

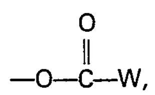

(V)

wherein W represents a group of general formula IV (wherein $R^3$, $R^4$, $R^5$ and $R^8$ are as hereinbefore defined) or a straight- or branched-chain alkyl group containing from 1 to 4 carbon atoms,

(B) when X represents an oxygen atom and $R^1$, $R^2$, $R^3$, $R^4$, $R^5$, $R^6$, $R^7$, $R^8$ and *m* are as defined in claim 1, the reaction of a compound of the general formula:—

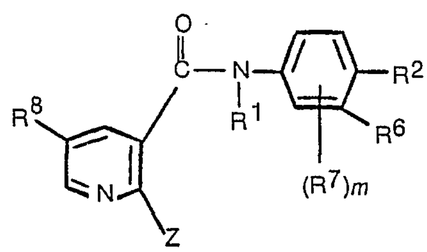

(VI)

(wherein Z represents a chlorine or bromine atom and $R^1$, $R^2$, $R^6$, $R^7$, $R^8$ and *m* are as defined in claim 1) with an alkali metal salt of a compound of the general formula:—

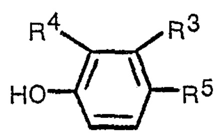

(VII)

wherein $R^3$, $R^4$ and $R^5$ are as defined in claim 1, or (C) when X represents a sulphur atom and $R^1$, $R^2$, $R^3$, $R^4$, $R^5$, $R^6$, $R^7$, $R^8$ and *m* are as defined in claim 1, the reaction of a compound of general formula I depicted in claim 1, wherein X represents an oxygen atom and $R^1$, $R^2$, $R^3$, $R^4$, $R^5$, $R^6$, $R^7$, $R^8$ and *m* are as defined in claim 1, with phosphorus pentasulphide.

6. A herbicidal composition which comprises, as active ingredient, at least one nicotinamide derivative of general formula I depicted in claim 1, wherein X, $R^1$, $R^2$, $R^3$, $R^4$, $R^5$, $R^6$, $R^7$, $R^8$ and *m* are as defined in claim 1, in association with one or more compatible herbicidally-acceptable diluents or carriers.

7. A herbicidal composition according to claim 6 in which the nicotinamide derivative incorporated in the composition is a compound claimed in claim 5.

8. A method of controlling the growth of weeds at a locus which comprises applying to the locus a herbicidally effective amount of a nicotinamide derivative claimed in claim 1 in a herbicidal composition as claimed in claim 6 or 7.

9. A method according to claim 8, in which the nicotinamide derivative is applied at a rate between 0.1 kg and 20 kg per hectare to an area used, or to be used, for growing a crop which is a cereal, preferably wheat, barley, oats, maize or rice, soya beans, field or dwarf beans, peas, lucerne, cotton, peanuts, flax, onions, carrots, cabbage, oilseed rape, sunflower, sugar beet, or permanent or sown grassland.

**Claims for the Contracting State: AT**

1. A process for the preparation of nicotinamide derivatives of the general formula:

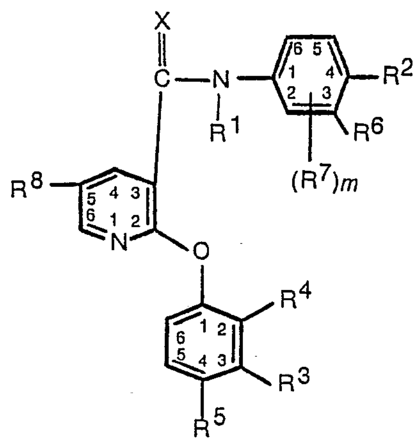

(I)

wherein X represents an oxygen or sulphur atom, $R^1$ represents a hydrogen atom or a methyl or ethyl group, $R^2$ represents a hydrogen or fluorine atom, a cyano group or a methyl or ethyl group optionally substituted by one or more fluorine atoms which is preferably trifluoromethyl, $R^3$ represents a fluorine, chlorine or bromine atom or a cyano, trifluoromethyl, methoxy, ethoxy, trifluoromethoxy or alkanesulphonyl group, wherein the alkane moiety of the alkanesulphonyl group may be straight- or branched-chain and contains from 1 to 4 carbon atoms, one of $R^4$ and $R^5$ represents a hydrogen atom and the other represents a hydrogen, fluorine, chlorine or bromine atom or a cyano, trifluoromethyl, methoxy, ethoxy, trifluoromethoxy or alkanesulphonyl group, wherein the alkane moiety of the alkanesulphonyl group may be straight- or branched-chain and contains from 1 to 4 carbon atoms, $R^6$ represents a hydrogen, fluorine or chlorine atom, $R^7$ represents a fluorine a chlorine atom, $R^8$ represents a hydrogen, fluorine, chlorine or bromine atom or a methyl or ethyl group and *m* represents 0, 1 or 2, wherein, when *m* represents 2, the atoms represented by the symbol $R^7$ may be the same or different, with the provisos (1) that when $R^2$ represents a hydrogen atom, $R^6$ represents a hydrogen atom and *m* represents 0, and (2) that when $R^2$ represents a fluorine atom, a cyano group or a methyl or ethyl group optionally substituted by one or more fluorine atoms, and $R^6$ represents a fluorine or chlorine atom, m represents 0 or 1 which process comprises:

(A) when X represents an oxygen atom and $R^1$, $R^2$, $R^3$, $R^4$, $R^5$, $R^6$, $R^7$, $R^8$ and *m* are as hereinbefore defined, the reaction of a compound of the general formula:—

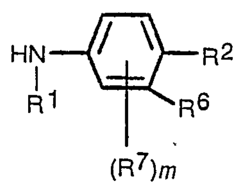

(II)

(wherein $R^1$, $R^2$, $R^6$, $R^7$ and *m* are as hereinbefore defined) or an acid addition salt thereof, with a compound of the general formula:—

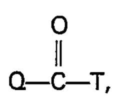

(III)

wherein Q represents a group of the general formula:—

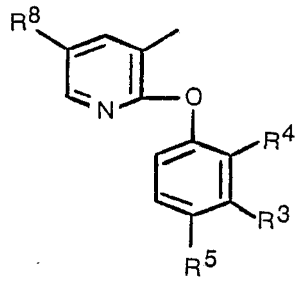

(IV)

(wherein $R^3$, $R^4$, $R^5$ and $R^8$ are as hereinbefore defined) and T represents a chlorine or bromine atom or a group of the general formula:—

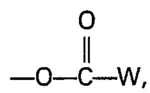

(V)

wherein W represents a group of general formula IV (wherein $R^3$, $R^4$, $R^5$ and $R^8$ are as hereinbefore defined) or a straight- or branched-chain alkyl group containing from 1 to 4 carbon atoms,

(B) when X represents an oxygen atom and $R^1$, $R^2$, $R^3$, $R^4$, $R^5$, $R^6$, $R^7$, $R^8$ and *m* are as hereinbefore defined, the reaction of a compound of the general formula:—

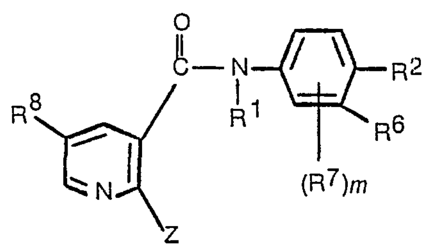

(VI)

(wherein Z represents a chlorine or bromine atom and $R^1$, $R^2$, $R^6$, $R^7$, $R^8$ and *m* are as hereinbefore defined) with an alkali metal salt of a compound of the general formula:—

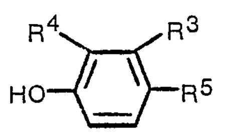

(VII)

wherein $R^3$, $R^4$ and $R^5$ are as hereinbefore defined, or (C) when X represents a sulphur atom and $R^1$, $R^2$, $R^3$, $R^4$, $R^5$, $R^6$, $R^7$, $R^8$ and *m* are as hereinbefore defined, the reaction of a compound of general formula I, wherein X represents an oxygen atom and $R^1$, $R^2$, $R^3$, $R^4$, $R^5$, $R^6$, $R^7$, $R^8$ and *m* are as hereinbefore defined, with phosphorus pentasulphide.

2. A herbicidal composition which comprises, as active ingredient, at least one nicotinamide derivative of general formula I depicted in claim 1, wherein X, $R^1$, $R^2$, $R^3$, $R^4$, $R^5$, $R^6$, $R^7$, $R^8$ and *m* are as defined in claim 1, in association with one or more compatible herbicidally-acceptable diluents or carriers.

3. A herbicidal composition according to claim 2 in which the nicotinamide derivative incorporated in the composition is a compound selected from N - (4 - fluorophenyl) - 2 - (3 - trifluoromethylphenoxy) - nicotinamide, N - phenyl - 2 - (3 - trifluoromethylphenoxy)nicotinamide, N - (2,4 - difluorophenyl) - 2 - (3 - trifluoromethylphenoxy)nicotinamide, N - (3,4 - difluorophenyl) - 2 - (3 - trifluoromethylphenoxy)-nicotinamide, N - (2,4,5 - trifluorophenyl) - 2 - (3 - trifluoromethylphenoxy)nicotinamide, N - (4 - fluorophenyl) - N - methyl - 2 - (3 - trifluoromethylphenoxy)nicotinamide, N - (4 - fluorophenyl) - 2 - (3 - trifluoromethylphenoxy)thionicotinamide, 2 - (3 - trifluoromethylphenoxy) - N - (2,4 - difluorophenyl) - 5 - methylnicotinamide and N - methyl - N - phenyl - 2 - (3 - trifluoromethylphenoxy)nicotinamide.

4. A method of controlling the growth of weeds at a locus which comprises applying to the locus a herbicidally effective amount of a nicotinamide derivative of general formula I depicted in claim 1, wherein X, $R^1$, $R^2$, $R^3$, $R^4$, $R^5$, $R^6$, $R^7$, $R^8$ and *m* are as defined in claim 1 in a herbicidal composition as claimed in claim 2 or 3.

5. A method according to claim 4, in which the nicotinamide derivative is applied at a rate between 0.1 kg and 20 kg per hectare at an area used, or to be used, for growing a crop which is a cereal, preferably wheat, barley, oats, maize or rice, soya beans, field or drawf beans, peas, lucerne, cotton, peanuts, flax, onions, carrots, cabbage, oilseed rape, sunflower, sugar beet, or permanent or sown grassland.

**Patentansprüche für die Vertragsstaaten: BE CH DE FR GB IT LI LU NL SE**

1. Nicotinamid-Derivate der allgemeinen Formel

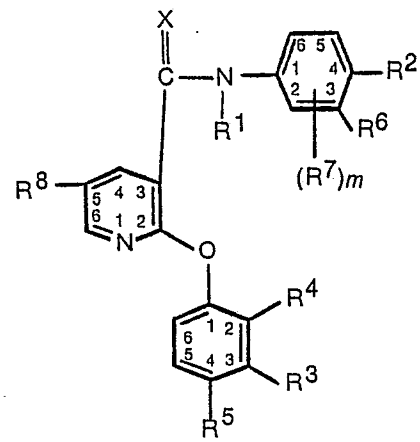

(I)

in der X ein Sauerstoffatom oder Schwefelatom, $R^1$ ein Wasserstoffatom oder eine Methyl- oder Äthylgruppe, $R^2$ ein Wasserstoff- oder Fluoratom eine Cyanogruppe oder eine Methylgruppe oder Äthylgruppe, gegebenenfalls substituiert durch ein oder mehrere Fluoratome, vorzugsweise die Trifluormethylgruppe, $R^3$ ein Fluor-, Chlor- oder Bromatom oder eine Cyano-, Trifluormethyl-, Methoxy-, Äthoxy-, Trifluormethoxy- oder Alkansulfonylgruppe bedeutet, wobei der Alkananteil der Alkansulfonylgruppe gerad- oder verzweigtkettig sein kann und 1 bis 4 Kohlenstoffatome enthält, einer der Reste $R^4$ und $R^5$ ein Wasserstoffatom und der andere ein Wasserstoff-, Fluor-, Chlor- oder Bromatom oder eine Cyano-, Trifluormethyl-, Methoxy-, Äthoxy-, Trifluormethoxy- oder Alkansulfonylgruppe bedeutet, wobei der Alkananteil der Alkansulfonylgruppe gerad- oder verzweigtkettig sein kann und 1 bis 4 Kohlenstoffatome enthält, $R^6$ ein Wasserstoff-, Fluor- oder Chloratom, $R^7$ ein Fluor- oder Chloratom, $R^8$ ein Wasserstoff-, Fluor-, Chlor- oder Bromatom oder eine Methyl- oder Äthylgruppe und m 0, 1 oder 2 bedeutet, wobei wenn m 2 bedeutet, die durch das Symbol $R^7$ angegebenen Atome gleich oder verschieden sein können, mit den Maßgaben, daß (1), wenn $R^2$ ein Wasserstoffatom bedeutet, $R^6$ ein Wasserstoffatom ist und (2) wenn $R^2$ ein Fluoratom,.eine Cyanogruppe oder eine Methyl- oder Äthylgruppe, gegebenenfalls substituiert durch ein oder mehrere Fluoratome, bedeutet, und $R^6$ ein Fluor- oder Chloratom ist, m 0 oder 1 bedeutet.

2. Nicotinamid-Derivate nach Anspruch 1, wobei $R^2$ ein Wasserstoff- oder Fluoratom, eine Cyanogruppe oder eine Methyl- oder Trifluormethylgruppe bedeutet, $R^3$ ein Fluor-, Chlor- oder Bromatom oder eine Methoxy-, Cyano-, Trifluormethyl-, Methansulfonyl- oder Äthansulfonylgruppe bedeutet, einer der Reste $R^4$ und $R^5$ ein Wasserstoffatom und der andere ein Wasserstoff-, Fluor- oder Chloratom bedeutet, $R^7$ ein Fluor- oder Chloratom bedeutet, $R^8$ ein Wasserstoffatom oder eine Methylgruppe bedeutet und X, $R^1$, $R^6$ und m die in Anspruch 1 angegebene Bedeutung haben.

3. Nicotinamid-Derivate nach Anspruch 1 oder 2, wobei X ein Sauerstoff- oder Schwefelatom bedeutet, $R^1$ ein Wasserstoffatom oder eine Methylgruppe bedeutet, $R^2$ ein Wasserstoff- oder Fluoratom bedeutet, $R^3$ eine Trifluormethylgruppe bedeutet, $R^4$ und $R^5$ jeweils ein Wasserstoffatom bedeuten, $R^6$ ein Wasserstoff- oder Fluoratom bedeutet, $R^7$ ein Fluoratom bedeutet, $R^8$ ein Wasserstoffatom oder eine Methylgruppe bedeutet und m 0 oder 1 bedeutet, mit den Maßgaben, daß (i), wenn $R^2$ ein Wasserstoffatom bedeutet, $R^6$ ein Wasserstoffatom bedeutet und m 0 ist und (ii), wenn $R^2$ ein Fluoratom bedeutet (a) $R^6$ ein Wasserstoffatom und m 0 bedeutet oder m 1 ist und $R^7$ ein Fluoratom in 2- (oder 6)- Stellung des Phenylringes ist oder (b) $R^6$ ein Fluoratom bedeutet und m 0 ist oder m 1 ist und $R^7$ ein Fluoratom in 6-Stellung des Phenylringes bedeutet.

4. N - (4 - Fluorphenyl) - 2 - (3 - trifluormethylphenoxy) - nicotinamid, N - Phenyl - 2 - (3 - trifluormethylphenoxy)nicotinamid, N - (2,4 - Difluorphenyl) - 2 - (3 - trifluormethylphenoxy) - nicotinamid, N - (3,4 - Difluorphenyl) - 2 - (3 - trifluormethylphenoxy)nicotinamid, N - (2,4,5 - Trifluorphenyl) - 2 - (3 - trifluormethylphenoxy)nicotinamid, N - (4 - Fluorphenyl) - N - methyl - 2 - (3 - trifluormethylphenoxy)-nicotinamid, N - (4 - Fluorphenyl) - 2 - (3 - trifluormethylphenoxy)thionicotinamid, 2 - (3 - Trifluormethylphenoxy) - N - (2,4 - difluorphenyl) - 5 - methylnicotinamid und N - Methyl - N - phenyl - 2 - (3 - trifluormethylphenoxy)nicotinamid.

5. Verfahren zur Herstellung von Nicotinamid-Derivaten der allgemeinen Formel I wie in Anspruch 1 angegeben, umfassend:

(A) wenn X ein Sauerstoffatom bedeutet und $R^1$, $R^2$, $R^3$, $R^4$, $R^5$, $R^6$, $R^7$, $R^8$ und m die in Anspruch 1 angegebene Bedeutung haben, die Umsetzung einer Verbindung der allgemeinen Formel

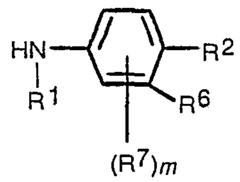

(II)

(wobei $R^1$, $R^2$, $R^6$, $R^7$ und m die oben angegebene Bedeutung haben) oder eines Säureadditionssalzes davon mit einer Verbindung der allgemeinen Formel

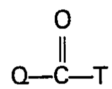

(III)

in der Q eine Gruppe der allgemeinen Formel

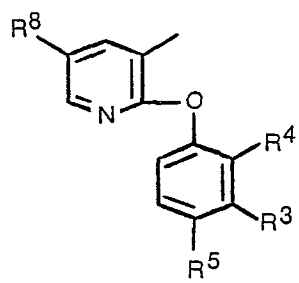

(IV)

ist, (in der $R^3$, $R^4$, $R^5$ und $R^8$ die oben angegebene Bedeutung haben) und T ein Chlor- oder Bromatom bedeutet oder eine Gruppe der allgemeinen Formel

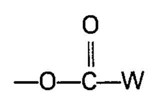

(V)

in der W eine Gruppe der allgemeinen Formel IV bedeutet, (wobei $R^3$, $R^4$, $R^5$ und $R^8$ die oben angegebene Bedeutung haben) oder eine gerad- oder verzweigtkettige Alkylgruppe mit 1 bis 4 Kohlenstoffatomen

(B) wenn X ein Sauerstoffatom bedeutet und $R^1$, $R^2$, $R^3$, $R^4$, $R^5$, $R^6$, $R^7$, $R^8$ und m die in Anspruch 1 angegebene Bedeutung haben, die Umsetzung einer Verbindung der allgemeinen Formel

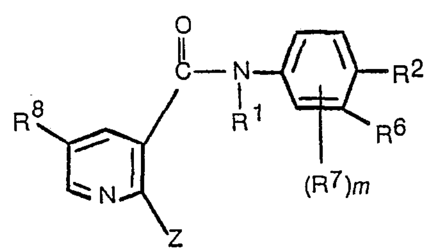

(VI)

(in der Z ein Chlor- oder Bromatom bedeutet und $R^1$, $R^2$, $R^6$, $R^7$, $R^8$ und m die in Anspruch 1 angegebene Bedeutung haben) mit einem Alkalisalz einer Verbindung der allgemeinen Formel

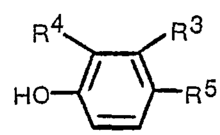

(VII)

in der $R^3$, $R^4$ und $R^5$ wie in Anspruch 1 definiert sind, oder (C) wenn X ein Schwefelatom bedeutet und $R^1$, $R^2$, $R^3$, $R^4$, $R^5$, $R^6$, $R^7$, $R^8$ und m wie in Anspruch 1 definiert sind, die Umsetzung einer Verbindung der allgemeinen Formel I wie in Anspruch 1 angegeben, wobei X ein Sauerstoffatom bedeutet und $R^1$, $R^2$, $R^3$, $R^4$, $R^5$, $R^6$, $R^7$, $R^8$ und m wie in Anspruch 1 definiert sind, mit Phosphorpentasulfid.

6. Herbizides Mittel, umfassend als Wirkstoff zumindest ein Nicotinamid-Derivat der allgemeinen Formel I wie in Anspruch 1 angegeben, wobei X, $R^1$, $R^2$, $R^3$, $R^4$, $R^5$, $R^6$, $R^7$, $R^8$ und m wie in Anspruch 1 definiert sind, zusammen mit einem oder mehreren verträglichen für das Herbizid annehmbaren Verdünnungsmitteln oder Trägern.

7. Herbizides Mittel nach Anspruch 6, in dem das in das Mittel eingebaute Nicotinamid-Derivat eine Verbindung wie in Anspruch 5 angegeben ist.

8. Verfahren zur Bekämpfung des Wachstums von Unkräutern an einer Stelle, umfassend das Aufbringen einer herbizid wirksamen Menge eines Nicotinamid-Derivats wie in Anspruch 1 angegeben, in einem herbiziden Mittel wie in Anspruch 6 oder 7 angegeben, auf die Stelle.

9. Verfahren nach Anspruch 8, wobei das Nicotinamid-Derivat in einer Menge zwischen 0,1 kg und 20 kg/ha auf einen Bereich, der angewandt worden ist oder werden soll für das Wachstum von Nutzpflanzen nämlich Getreide, vorzugsweise Weizen, Gerste, Hafer, Mais oder Reis, Sojabohnen, Feld- oder Zwergbohnen, Erbsen, Luzerne, Baumwolle, Erdnüssen, Flachs, Zwiebeln, Karotten, Kohl, Raps, Sonnenblumen, Zuckerrüben oder permanentem oder ausgesätem Grasland, aufgebracht wird.

**Patentansprüche für den Vertragsstaat: AT**

1. Verfahren zur Herstellung eines Nicotinamid-Derivats der allgemeinen Formel

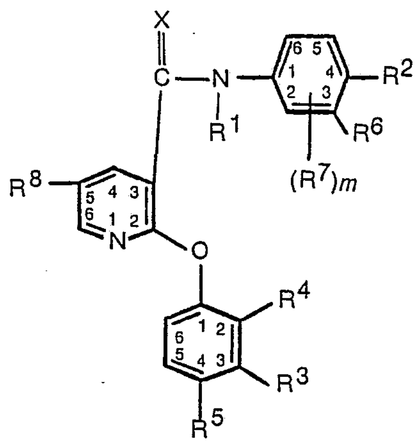

(I)

in der X ein Sauerstoffatom oder Schwefelatom, $R^1$ ein Wasserstoffatom oder eine Methyl- oder Äthylgruppe, $R^2$ ein Wasserstoff- oder Fluoratom oder eine Cyanogruppe oder eine Methylgruppe oder Äthylgruppe, gegebenenfalls substituiert durch ein oder mehrere Fluoratome, vorzugsweise die Trifluormethylgruppe, $R^3$ ein Fluor-, Chlor- oder Bromatom oder eine Cyano-, Trifluormethyl-, Methoxy-, Äthoxy-, Trifluormethoxy- oder Alkansulfonylgruppe bedeutet, wobei der Alkananteil der Alkansulfonylgruppe gerad- oder verzweigtkettig sein kann und 1 bis 4 Kohlenstoffatome enthält, einer der Reste $R^4$ und $R^5$ ein Wasserstoffatom und der andere ein Wasserstoff-, Fluor-, Chlor- oder Bromatom oder eine Cyano-, Trifluormethyl-, Methoxy-, Äthoxy-, Trifluormethoxy- oder Alkansulfonylgruppe bedeutet, wobei der Alkananteil der Alkansulfonylgruppe gerad- oder verzweigtkettig sein kann und 1 bis 4 Kohlenstoffatome enthält, $R^6$ ein Wasserstoff-, Fluor- oder Chloratom, $R^7$ ein Fluor- oder Chloratom, $R^8$ ein Wasserstoff-, Fluor-, Chlor- oder Bromatom oder eine Methyl- oder Äthylgruppe und m 0, 1 oder 2 bedeutet, wobei, wenn m 2 bedeutet, die durch das Symbol $R^7$ angegebenen Atome gleich oder verschieden sein können, mit den Maßgaben, daß (1), wenn $R^2$ ein Wasserstoffatom bedeutet, $R^6$ ein Wasserstoffatom ist und (2), wenn $R^2$ ein Fluoratom, eine Cyanogruppe oder eine Methyl- oder Äthylgruppe, gegebenenfalls substituiert durch ein oder mehrere Fluoratome, bedeutet, und $R^6$ ein Fluor- oder Chloratom ist, m 0 oder 1 bedeutet, umfassend

(A) wenn X ein Sauerstoffatom bedeutet und $R^1$, $R^2$, $R^3$, $R^4$, $R^5$, $R^6$, $R^7$, $R^8$ und m die oben angegebene Bedeutung haben die Umsetzung einer Verbindung der allgemeinen Formel

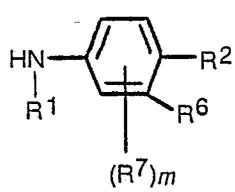

(II)

(wobei $R^1$, $R^2$, $R^6$, $R^7$ und m die oben angegebene Bedeutung haben) oder eines Säureadditionssalzes davon mit einer Verbindung der allgemeinen Formel

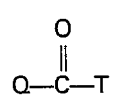

(III)

in der Q eine Gruppe der allgemeinen Formel

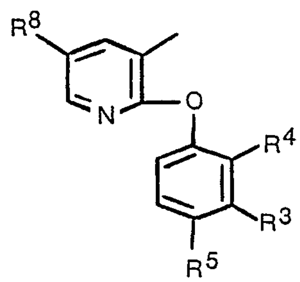

(IV)

ist, (in der $R^3$, $R^4$, $R^5$ und $R^8$ die oben angegebene Bedeutung haben) und T ein Chlor- oder Bromatom bedeutet, oder eine Gruppe der allgemeinen Formel

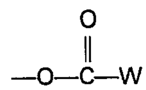

(V)

in der W eine Gruppe der allgemeinen Formel IV bedeutet, (wobei $R^3$, $R^4$, $R^5$ und $R^8$ die oben angegebene Bedeutung haben) oder eine gerad- oder verzweigtkettige Alkylgruppe mit 1 bis 4 Kohlenstoffatomen,

(B) wenn X ein Sauerstoffatom bedeutet und $R^1$, $R^2$, $R^3$, $R^4$, $R^5$, $R^6$, $R^7$, $R^8$ und m die oben angegebene Bedeutung haben, die Umsetzung einer Verbindung der allgemeinen Formel

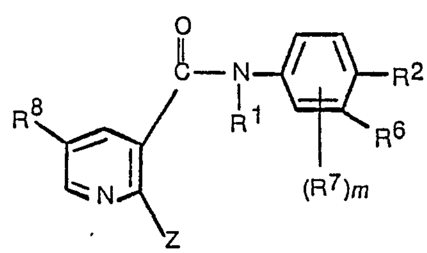

(VI)

(in der Z ein Chlor- oder Bromatom bedeutet und $R^1$, $R^2$, $R^6$, $R^7$, $R^8$ und m die oben angegebene Bedeutung haben) mit einem Alkalisalz einer Verbindung der allgemeinen Formel

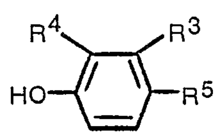

(VII)

in der $R^3$, $R^4$ und $R^5$ wie oben definiert sind, oder (C) wenn X ein Schwefelatom bedeutet und $R^1$, $R^2$, $R^3$, $R^4$, $R^5$, $R^6$, $R^7$, $R^8$ und m wie oben definiert sind, die Umsetzung einer Verbindung der allgemeinen Formel I wobei X ein Sauerstoffatom bedeutet und $R^1$, $R^2$, $R^3$, $R^4$, $R^5$, $R^6$, $R^7$, $R^8$ und m wie oben definiert sind mit Phosphorpentasulfid.

2. Herbizides Mittel, umfassend als Wirkstoff zumindest ein Nicotinamid-Derivat der allgemeinen Formel I wie in Anspruch 1 angegeben, wobei X, $R^1$, $R^2$, $R^3$, $R^4$, $R^5$, $R^6$, $R^7$, $R^8$ und m wie in Anspruch 1 definiert sind, zusammen mit einem oder mehreren verträglichen für das Herbizid annehmbaren Verdünnungsmitteln oder Trägern.

3. Herbizides Mittel, bei dem das in das Mittel eingebaute Nicotinamid-Derivat eine Verbindung ausgewählt aus N - (4 - Fluorphenyl) - 2 - (3 - trifluormethylphenoxy) - nicotinamid, N - Phenyl - 2 - (3 - trifluormethylphenoxy)nicotinamid, N - (2,4 - Difluorphenyl) - 2 - (3 - trifluormethylphenoxy) - nicotinamid, N - (3,4 - Difluorphenyl) - 2 - (3 - trifluormethylphenoxy)nicotinamid, N - (2,4,5 - Trifluorphenyl) - 2 - (3 - trifluormethylphenoxy)nicotinamid, N - (4 - Fluorphenyl) - N - methyl - 2 - (3 - trifluormethylphenoxy)nicotinamid, N - (4 - Fluorphenyl) - 2 - (3 - trifluormethylphenoxy)thionicotinamid, 2 - (3 - Trifluormethylphenoxy) - N - (2,4 - difluorphenyl) - 5 - methylnicotinamid und N - Methyl - N - phenyl - 2 - (3 - trifluormethylphenoxy)nicotinamid.

4. Verfahren zur Bekämpfung des Wachstums von Unkräutern an einer Stelle, umfassend das Aufbringen einer herbizid wirksamen Menge eines Nicotinamid-Derivats der allgemeinen Formel I wie in Anspruch 1 angegeben, in einem herbiziden Mittel wie in Anspruch 2 oder 3 angegeben, wobei X, $R^1$, $R^2$, $R^3$, $R^4$, $R^5$, $R^6$, $R^7$, $R^8$ und m wie in Anspruch 1 definiert sind auf die Stelle.

5. Verfahren nach Anspruch 4, wobei das Nicotinamid-Derivat in einer Menge zwischen 0,1 kg und 20 kg/ha auf einen Bereich, der angewandt worden ist oder werden soll für das Wachstum von Nutzpflanzen, nämlich Getreide, vorzugsweise Weizen, Gerste, Hafer, Mais oder Reis, Sojabohnen, Feld- oder

Zwergbohnen, Erbsen, Luzerne, Baumwolle, Erdnüssen, Flachs, Zwiebeln, Karotten, Kohl, Raps, Sonnenblumen, Zuckerrüben oder permanentem oder ausgesätem Grasland, aufgebracht wird.

**Revendications pour les Etats contractants: BE CH DE FR GB IT LI LU NL SE**

1. Dérivés de nicotinamide de formule générale:

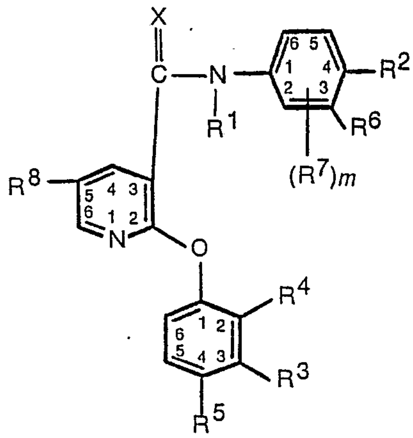

(I)

dans laquelle:

X représente un atome d'oxygène ou de soufre;

$R^1$ représente un atome d'hydrogène ou un groupe méthyle ou éthyle;

$R^2$ représente un atome d'hydrogène ou de fluor, un groupe cyano ou un groupe méthyle ou éthyle éventuellement substitué par un ou plusieurs atomes de fluor et constituant de préférence le radical trifluorométhyle;

$R^3$ représente un atome de fluor, de chlore ou de brome ou un groupe cyano, trifluorométhyle, méthoxy, éthoxy, trifluorométhoxy ou alcanesulfonyle, dans lequel la partie alcane du groupe alcane sulfonyle peut être linéaire ou ramifiée et comprend de 1 à 4 atomes de carbone;

l'un des radicaux $R^4$ et $R^5$ représente un atome d'hydrogène et l'autre représente un atome d'hydrogène, de fluor, de chlore ou de brome ou un groupe cyano, trifluorométhyle, méthoxy, éthoxy, trifluorométhoxy ou alcanesulfonyle, dans lequel la partie alcane du groupe alcane sulfonyle peut être linéaire ou ramifié et comprend de 1 à 4 atomes de carbone;

$R^6$ représente un atome d'hydrogène, de fluor ou de chlore;

$R^7$ représente un atome de fluor ou de chlore;

$R^8$ représente un atome d'hydrogène, de fluor, de chlore ou de brome ou un groupe méthyle ou éthyle;

m est égal à 0,1 ou 2;

lorsque m est égal à 2, les atomes représentés par $R^7$ peuvent être identiques ou différents, sous réserves que;

(1) quand $R^2$ représente l'atome d'hydrogène, $R^6$ représente l'atome d'hydrogène et m est nul, et,

(2) quand $R^2$ représente l'atome de fluor, un groupe cyano ou un groupe méthyle ou éthyle éventuellement substitué par un ou plusieurs atomes de fluor, et $R^6$ représente un atome de fluor ou de chlore, alors m est égal à 0 ou 1.

2. Dérivés de nicotinamide selon la revendication 1, dans lesquels:

$R^2$ représente un atome d'hydrogène ou de fluor, un groupe cyano ou un groupe méthyle ou trifluorométhyle;

$R^3$ représente un atome de fluor, de chlore ou de brome ou un groupe méthoxy, cyano, trifluorométhyle, méthanesulfonyle ou éthanesulfonyle;

l'un des radicaux $R^4$ et $R^5$ représente l'atome d'hydrogène et l'autre représente l'atome d'hydrogène, de fluor ou de chlore;

$R^7$ représente un atome de fluor ou de chlore;

$R^8$ représente un atome d'hydrogène ou un groupe méthyle;

X, $R^1$, $R^6$ et m sont tels que définis dans la revendication 1.

3. Dérivés de Nicotinamide selon les revendications 1 ou 2 dans lesquels:

X est un atome d'oxygène ou de soufre;

$R^1$ représente un atome d'hydrogène ou un groupe méthyle;

$R^2$ représente un atome d'hydrogène ou de fluor;

$R^3$ représente un groupe trifluorométhyle;

$R^4$ et $R^5$ sont chacun l'atome d'hydrogène;

$R^6$ représente un atome d'hydrogène ou de fluor;

$R^7$ représente un atome de fluor;

$R^8$ représente un atome d'hydrogène ou un groupe méthyle;
m est 0 ou 1,
sous réserves que:
(i) quand $R^2$ est l'atome d'hydrogène, alors $R^6$ est l'atome d'hydrogène et m est nul, et
(ii) quand $R^2$ est l'atome de fluor
a) $R^6$ est l'atome d'hydrogène et m est nul ou m est égal à 1 et $R^7$ est l'atome de fluor en position 2 ou 6 du noyau phényle, ou
b) $R^6$ est l'atome de fluor et m est nul, ou m est 1 et $R^7$ est l'atome de fluor en position 6 du noyau phényle.

4. Le (Trifluorométhyl-3 phénoxy)-2,N-(fluoro-4 phényl) nicotinamide, (Trifluorométhyl-3 phénoxy)-2,N-phényl-nicotinamide, (Trifluorométhyl-3 phénoxy)-2,N-(difluoro-2,4 phényl)nicotinamide, (Trifluorométhyl-3 phénoxy)-2,N-(difluoro-3,4 phényl) nicotinamide, (Trifluorométhyl-3 phénoxy)-2,N-(trifluoro-2,4,5-phényl) nicotinamide, (Trifluorométhyl-3 phénoxy)-2,N-(fluoro-4 phényl)-N-(méthyl) nicotinamide, (Trifluorométhyl-3 phénoxy)-2,N-(fluoro-4 phényl)-thionicotinamide, Méthyl-5-(trifluorométhyl-3 phénoxy)-2,N-(difluoro-2,4 phényl) nicotinamide, (Trifluorométhyl-3 phénoxy)2,N-phényl-N-méthyl nicotinamide.

5. Procédé de préparation de dérivés de nicotinamide de formule générale (I) décrite dans la revendication 1 qui comprend

A) quand X est l'atome d'oxygène et $R^1$, $R^2$, $R^3$, $R^4$, $R^5$, $R^6$, $R^7$, $R^8$ et m sont tels que définis dans la revendication 1, la réaction d'un composé de formule générale

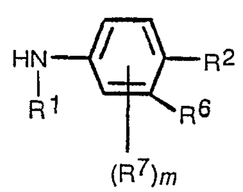

(II)

(dans laquelle $R^1$, $R^2$, $R^6$, $R^7$ et m sont tels que définis ci-avant) ou d'un sel par addition d'acide d'un tel composé, avec un composé de formule générale

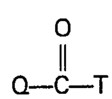

(III)

dans laquelle Q représente un groupe de formule générale

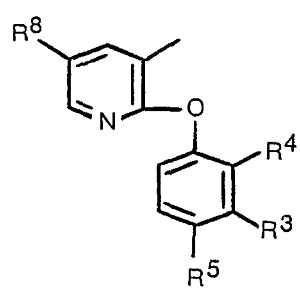

(IV)

(dans laquelle $R^3$, $R^4$, $R^5$ et $R^8$ sont tels que définis ci-avant) et T est un atome de chlore ou de brome ou un groupe de formule générale:

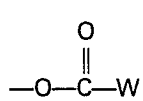

(V)

dans laquelle W est un groupe de formule générale IV (dans laquelle $R^3$, $R^4$, $R^5$ et $R^8$ sont tels que définis ci-avant) ou un groupe alkyle linéaire ou ramifié contenant de 1 à 4 atomes de carbone,

B) quand X est l'atome d'oxygène et $R^1$, $R^2$, $R^3$, $R^4$, $R^5$, $R^6$, $R^7$, $R^8$ et m sont tels que définis dans la revendication 1, la réaction d'un composé de formule générale:

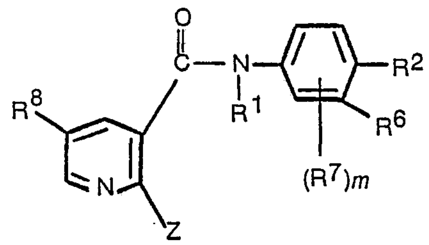

(VI)

(dans laquelle Z est un atome de chlore ou de brome et $R^1$, $R^2$, $R^6$, $R^7$, $R^8$ et m sont tels que définis dans la revendication 1) avec un sel de métal alcalin d'un composé de formule générale:

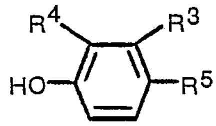

(VII)

dans laquelle $R^3$, $R^4$ et $R^5$ sont tels que définis dans la revendication 1, ou

C) quand X représente l'atome de soufre et $R^1$, $R^2$, $R^3$, $R^4$, $R^5$, $R^6$, $R^7$, $R^8$ et m sont tels que définis dans la revendication 1, la réaction avec du pentasulfure de phosphore d'un composé de formule générale (I) décrite dans la revendication 1, dans laquelle X représente l'atome d'oxygène et $R^1$, $R^2$, $R^3$, $R^4$, $R^5$, $R^6$, $R^7$, $R^8$ et m sont tels que définis dans la revendication 1.

6. Une composition herbicide qui comprend, comme matière active, au moins un dérivé de nicotinamide de formule générale (I) décrite dans la revendication 1, dans laquelle X, $R^1$, $R^2$, $R^3$, $R^4$, $R^5$, $R^6$, $R^7$, $R^8$ et m sont tels que définis dans la revendication 1, en association avec un ou plusieurs diluants ou support compatibles et acceptables pour une application herbicide.

7. Une composition herbicide selon la revendication 6 dans laquelle le dérivé de nicotinamide incorporé dans la composition est un composé revendiqué dans la revendication 5.

8. Méthode de contrôle de la croissance des mauvaises herbes en un endroit donné qui comprend l'application en cet endroit d'une quantité efficace du point de vue herbicide d'un dérivé de nicotinamide revendiqué dans la revendication 1 dans une composition herbicide telle que revendiquée dans l'une des revendications 6 ou 7.

9. Méthode selon la revendication 8 dans laquelle le dérivé de nicotinamide est appliqué à une dose comprise entre 0,1 et 20 kg/ha sur une surface utilisée ou devant être utilisée pour cultiver une culture qui est un céréale, de préférence le blé, l'orge, les avoines, le maïs ou le riz, le soja, les haricots, les haricots nains, les pois, la luzerne, le cotonnier, les arachides, l'ail, les oignons, les carottes, les choux, le colza, le tournesol, la betterave à sucre ou l'herbage permanent ou semé.

**Revendications pour l'Etat contractant: AT**

1. Procédé de préparation de dérivés de nicotinamide de formule générale:

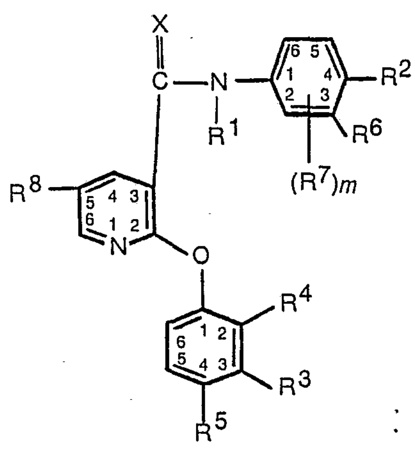

(I)

dans laquelle:

X représente un atome d'oxygène ou de soufre;

$R^1$ représente un atome d'hydrogène ou un groupe méthyle ou éthyle;

$R^2$ représente un atome d'hydrogène ou de fluor, un groupe cyano ou un groupe méthyle ou éthyle éventuellement substitué par un ou plusieurs atomes de fluor et constitué de préférence par le radical trifluorométhyle;

$R^3$ représente un atome de fluor, de chlore ou de brome ou un groupe cyano, trifluorométhyle, méthoxy, éthoxy, trifluorométhoxy ou alcanesulfonyle, dans lequel la partie alcane du groupe alcane sulfonyle peut être linéaire ou ramifiée et comprend de 1 à 4 atomes de carbone;

l'un des radicaux $R^4$ et $R^5$ représente un atome d'hydrogène et l'autre représente un atome d'hydrogène, de fluor, de chlore ou de brome ou un groupe cyano, trifluorométhyle, méthoxy, éthoxy, trifluorométhoxy ou alcanesulfonyle, dans lequel la partie alcane du groupe alcane sulfonyle peut être linéaire ou ramifiée et comprend de 1 à 4 atomes de carbone;

$R^6$ représente un atome d'hydrogène, de fluor ou de chlore;

$R^7$ représente un atome de fluor ou de chlore;

$R^8$ représente un atome d'hydrogène, de fluor, de chlore ou de brome ou un groupe méthyle ou éthyle;

m est égal à 0,1 ou 2;

lorsque m est égal à 2, les atomes représentés par $R^7$ peuvent être identiques ou différents, sous réserves que:

(1) quand $R^2$ représente l'atome d'hydrogène, $R^6$ représente l'atome d'hydrogène et m est nul, et,

(2) quand $R^2$ représente l'atome de fluor, un groupe cyano ou un groupe méthyle ou éthyle éventuellement substitué par un ou plusieurs atomes de fluor, et $R^6$ représente un atome de fluor ou de chlore, alors m est égal à 0 ou 1, ledit procédé comprenant:

A) quand X est l'atome d'oxygène et $R^1$, $R^2$, $R^3$, $R^4$, $R^5$, $R^6$, $R^7$, $R^8$ et m sont tels que définis ci-avant, la réaction d'un composé de formule générale:

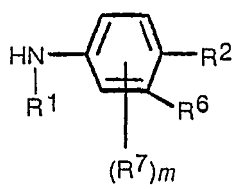

(II)

(dans laquelle $R^1$, $R^2$, $R^6$, $R^7$ et m sont tels que définis ci-avant) ou d'un sel par addition d'acide d'un tel composé, avec un composé de formule générale

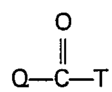

(III)

dans laquelle Q représente un groupe de formule générale

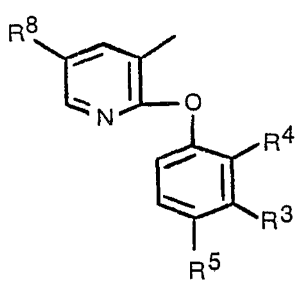

(IV)

(dans laquelle $R^3$, $R^4$, $R^5$ et $R^8$ sont tels que définis ci-avant) et T est un atome de chlore ou de brome ou un groupe de formule générale:

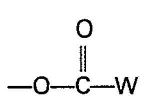

(V)

dans laquelle W est un groupe de formule générale IV (dans laquelle $R^3$, $R^4$, $R^5$ et $R^8$ sont tels que définis ci-avant) ou un groupe alkyle linéaire ou ramifié contenant de 1 à 4 atomes de carbone,

B) quand X est l'atome d'oxygène et $R^1$, $R^2$, $R^3$, $R^4$, $R^5$, $R^6$, $R^7$, $R^8$ et m sont tels que définis dans la revendication 1, la réaction d'un composé de formule générale:

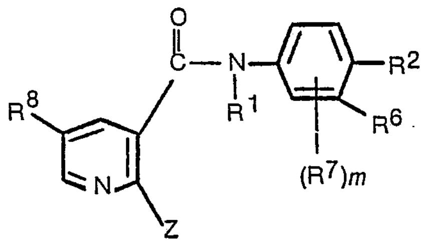

(VI)

(dans laquelle Z est un atome de chlore ou de brome et $R^1$, $R^2$, $R^6$, $R^7$, $R^8$ et m sont tels que définis ci-avant) avec un sel de métal alcalin d'un composé de formule générale:

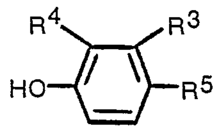

(VII)

dans laquelle $R^3$, $R^4$ et $R^5$ sont tels que définis ci-avant, ou

C) quand X représente l'atome de soufre et $R^1$, $R^2$, $R^3$, $R^4$, $R^5$, $R^6$, $R^7$, $R^8$ et m sont tels que définis dans la revendication 1, la réaction avec du pentasulfure de phosphore d'un composé de formule générale (I), dans laquelle X représente l'atome d'oxygène et $R^1$, $R^2$, $R^3$, $R^4$, $R^5$, $R^6$, $R^7$, $R^8$ et m sont tels que définis ci-avant.

2. Une composition herbicide qui comprend, comme matière active, au moins un dérivé de nicotinamide de formule générale (I) décrite dans la revendication 1, dans laquelle X, $R^1$, $R^2$, $R^3$, $R^4$, $R^5$, $R^6$, $R^7$, $R^8$ et m sont tels que définis dans la revendication 1, en association avec un ou plusieurs diluants ou support compatibles et acceptables pour une application herbicide.

3. Une composition herbicide selon la revendication 2 dans laquelle le dérivé de nicotinamide incorporé à la composition est un composé choisi parmi le (Trifluorométhyl-3 phénoxy)-2,N-(fluoro-4 phényl) nicotinamide, (Trifluorométhyl-3 phénoxy)-2,N-phényl-nicotinamide, (Trifluorométhyl-3 phénoxy)-2,N-(difluoro-2,4 phényl)nicotinamide, (Trifluorométhyl-3 phénoxy)-2,N-(difluoro-3,4 phényl) nicotinamide, (Trifluorométhyl-3 phénoxy)-2,N-(trifluoro-2,4,5-phényl) nicotinamide, (Trifluorométhyl-3 phénoxy)-2,N-(fluoro-4 phényl)-N-(méthyl) nicotinamide, (Trifluorométhyl-3 phénoxy)-2,N-(fluoro-4 phényl)-thionicotinamide, Méthyl-5-(trifluorométhyl-3 phénoxy)-2,N-(difluoro-2,4 phényl) nicotinamide, (Trifluorométhyl-3 phénoxy)2,N-phényl-N-méthyl nicotinamide.

4. Méthode de contrôle de la croissance des mauvaises herbes en un endroit donné qui comprend l'application en cet endroit d'une quantité efficace du point de vue herbicide d'un dérivé de nicotinamide de formule générale (I) décrite dans la revendication 1 dans laquelle X, $R^1$, $R^2$, $R^3$, $R^4$, $R^5$, $R^6$, $R^7$, $R^8$ et m sont tels que définis dans la revendication 1, dans une composition herbicide telle que revendiquée dans l'une des revendications 2 ou 3.

5. Methode selon la revendication 4 dans laquelle le dérivé de nicotinamide est appliqué à une dose comprise entre 0,1 et 20 kg/ha sur une surface utilisée ou devant être utilisée pour cultiver une culture qui est une céréale, de préférence le blé, l'orge, les avoines, le maïs ou le riz, le soja, les haricots, les haricots nains, les pois, la luzerne, le cotonnier, les arachides, l'ail, les oignons, les carottes, les choux, le colza, le tournesol, la betterave à sucre ou l'herbage permanent ou semé.